# EUROPÄISCHE PATENTSCHRIFT

(11) **EP 3 411 085 B1**
(45) Veröffentlichungstag und Bekanntmachung des Hinweises auf die Patenterteilung: **26.01.2022**
(21) Anmeldenummer: 17703727.2
(22) Anmeldetag: 03.02.2017
(51) Int. Cl.: A61L 2/14, A61N 1/44, A61C 19/06, A61C 17/02, A61C 17/00

(54) **VORRICHTUNG UND VERFAHREN ZUM BEHANDELN VON GEGENSTÄNDEN, INSBESONDERE VON ZAHNPROTHESEN UND/ODER ZÄHNEN**
DEVICE AND METHOD FOR TREATING OBJECTS, IN PARTICULAR DENTAL PROSTHETICS AND/OR TEETH
DISPOSITIF ET PROCÉDÉ POUR TRAITER DES OBJETS, NOTAMMENT DES PROTHÈSES DENTAIRES ET/OU DES DENTS

(30) Priorität: 05.02.2016 DE 102016201818
(43) Veröffentlichungstag der Anmeldung: 12.12.2018
(73) Patentinhaber: terraplasma GmbH, 85748 Garching (DE)
(72) Erfinder: MORFILL, Gregor, 81927 München (DE); ZIMMERMANN, Julia, 81925 München (DE); SHIMIZU, Tetsuji, Ibaraki 305-0044 (JP); LI, Yangfang, 82140 Olching (DE); BINDER, Sylvia, 85560 Ebersberg (DE); CANTZLER, Maximilian, 85560 Ebersberg (DE); WEILEMANN, Hannes, 81925 München (DE)
(74) Vertreter: Kordel, Mattias
(86) Internationale Anmeldenummer: PCT/EP2017/052425
(87) Internationale Veröffentlichungsnummer: WO 2017/134243

(56) Entgegenhaltungen:
- WO-A1-2011/123124
- JP-A- 2008 302 198
- KR-B1- 100 918 474
- US-A1- 2013 272 929

## Beschreibung

Die Erfindung betrifft eine Vorrichtung sowie ein Verfahren zum Behandeln, insbesondere Reinigen, von Gegenständen, insbesondere von Zahnprothesen, außerhalb des Mundes.

Zur Reinigung von Zahnprothesen - insbesondere außerhalb des Mundes - werden typischerweise chemische Lösungen eingesetzt, die beispielsweise mittels in Wasser aufzulösenden Brausetabletten bereitgestellt werden. Dabei werden vergleichsweise hohe Konzentrationen von Reinigungschemikalien eingesetzt, und die Reinigungslösungen sind typischerweise nicht für den Verzehr geeignet, sodass es auch eines intensiven Spülens einer derart gereinigten Zahnprothese nach der Reinigung bedarf, bevor diese wieder in den Mund eingesetzt werden kann. Auch zum Reinigen von Zähnen werden typischerweise Chemikalien eingesetzt - insbesondere in Form von Zahnpasta - die nicht geschluckt werden sollten. Zum Bleichen von Zahnprothesen und/oder Zähnen werden typischerweise Konzentrationen von Wasserstoffperoxid verwendet, die weit oberhalb eines in der Europäischen Union geltenden Grenzwerts für nicht-professionelles Zahnbleichen oder für Trinkwasser liegen. Entsprechende Präparationen sind daher nicht oder nur durch geschultes Personal direkt im Mund anwendbar, und es ergibt sich mit Bezug auf die Trinkbarkeit einer Bleichlösung für Zahnprothesen das gleiche Problem, welches zuvor bereits für Reinigungslösungen geschildert wurde. Hinzu kommt, dass die zuvor beschriebenen Reinigungs- und/oder Bleichlösungen aufwendig zu fertigen sind, wobei stets auf zusätzlich zu beschaffende Chemikalien zurückgegriffen werden muss.

Aus der amerikanischen Offenlegungsschrift US 2013/272929 A1 ist eine Sterilisationsstation mit einer Flüssigkeitsquelle und einem oder mehreren Plasmageneratoren zur Erzeugung von einem nicht-thermischen Plasma bekannt. Eine oder mehrere Düsen sprühen einen Nebel oder Flüssigkeitsstrom durch das von einem oder mehreren Plasmageneratoren erzeugte Plasma, um die Flüssigkeit zu aktivieren. Die Flüssigkeit wird dann zur Sterilisation eines Objekts verwendet. Eine weitere Sterilisationsstation umfasst eine Kammer zur Aufnahme einer Flüssigkeit und einen Plasmagenerator, der mit der Kammer zur Erzeugung von Plasma in Verbindung steht. Eine zirkulierende Quelle bewegt die Flüssigkeit in der Kammer an dem vom Plasmagenerator erzeugten Plasma vorbei, um die Flüssigkeit zu aktivieren, und eine oder mehrere Sprühdüsen beschichten die Oberfläche eines Objekts mit der Flüssigkeit, die durch das Plasma aktiviert wird.

Der Erfindung liegt die Aufgabe zugrunde, eine Vorrichtung und ein Verfahren zur Behandlung, insbesondere zur Reinigung, von Gegenständen, insbesondere von Zahnprothesen zu schaffen, wobei die genannten Nachteile nicht auftreten.

Die Aufgabe wird gelöst, indem die Gegenstände der unabhängigen Ansprüche geschaffen werden. Vorteilhafte Ausgestaltungen ergeben sich aus den Unteransprüchen.

Die Aufgabe wird insbesondere gelöst, indem eine Vorrichtung zur Behandlung, insbesondere zur Reinigung, von Gegenständen, insbesondere von Zahnprothesen und/oder Zähnen geschaffen wird, welche ein Aufnahmevolumen zur Aufnahme eines Reinigungsfluids aufweist, wobei die Vorrichtung weiter eine Plasmaquelle aufweist, die eingerichtet ist zur Erzeugung eines nicht-thermischen Plasmas. Die Vorrichtung ist eingerichtet zur Durchmischung eines Plasmaerzeugnisses des nicht-thermischen Plasmas mit dem Reinigungsfluid, wodurch ein aktiviertes Reinigungsfluid erzeugbar ist. Weiterhin ist die Vorrichtung eingerichtet zur Anwendung des aktivierten Reinigungsfluids auf einen Gegenstand, insbesondere auf wenigstens eine Zahnprothese und/oder wenigstens einen Zahn.

Wo hier und im Folgenden auf Zahnprothesen und/oder Zähne Bezug genommen ist, sind stets diese spezifischen Ausführungsbeispiele, aber in analoger Weise auch andere Gegenstände angesprochen, auf die das aktivierte Reinigungsfluid in entsprechender Weise angewendet werden kann.

Mittels der Plasmaquelle ist ein nicht-thermisches Plasma ohne Rückgriff auf zusätzlich beschaffte Chemikalien bereitstellbar, wobei es insbesondere dann keines Rückgriffs auf zusätzliche Chemikalien bedarf, wenn als Reinigungsfluid Wasser verwendet wird. Das aktivierte Reinigungsfluid, welches letztlich als Agens zur Behandlung von Zahnprothesen und/oder Zähnen dient, ist daher einfach, unkompliziert, schnell und kostengünstig bereitstellbar. Es zeigt sich darüber hinaus, dass mittels einer Plasmaaktivierung eines Reinigungsfluids, insbesondere von Wasser, ein einerseits hoch wirksames und andererseits mit Trinkwassernormen kompatibles Reinigungsfluid bereitgestellt werden kann, welches ohne weiteres auch direkt im Mund verwendbar ist und geschluckt werden darf, wobei auch eine außerhalb des Mundes gereinigte Zahnprothese nicht in gleicher Weise umfangreich gespült werden muss, wie dies bei herkömmlichen, chemischen Reinigungslösungen der Fall ist, da das aktivierte Reinigungsfluid Trinkwasserqualität aufweisen kann.

Unter einer Behandlung von Gegenständen, insbesondere von Zahnprothesen wird hier insbesondere eine Reinigung, ganz besonders eine Desinfektion und insbesondere eine Reduzierung einer Beladung von Gegenständen, insbesondere von Zahnprothesen mit Bakterien und/oder Viren, verstanden, sowie zusätzlich oder alternativ ein Bleichen von Zahnprothesen. Insbesondere ist die Behandlung bevorzugt keine medizinische Behandlung, sondern eine im Rahmen der täglichen Zahn- oder Zahnprothesenhygiene durchzuführende Behandlung, mithin insbesondere eine prophylaktische oder kosmetische Behandlung.

Die Erfindung betrifft also insbesondere eine Vorrichtung und ein Verfahren zur hygienischen und/oder kosmetischen Behandlung von Gegenständen, insbesondere von Zahnprothesen.

Unter einem Aufnahmevolumen wird insbesondere ein räumlicher Bereich der Vorrichtung verstanden, der geeignet ist, um ein Reinigungsfluid aufzunehmen. Dabei wird unter "Aufnehmen" sowohl ein statisches Halten als auch ein Durchleiten des Reinigungsfluids durch das Aufnahmevolumen verstanden. Bevorzugt ist das Aufnahmevolumen als geschlossenes Volumen ausgebildet oder derart vorgesehen, dass es durch eine Abdeckeinrichtung schließbar ist, sodass mittels der Abdeckeinrichtung ein geschlossenes Volumen bereitgestellt werden kann.

Unter einem Reinigungsfluid wird insbesondere ein fluides Medium verstanden, welches als Trägersubstanz, insbesondere als Lösungsmittel, für in dem nicht-thermischen Plasma und/oder dem Plasmaerzeugnis vorhandene aktive Spezies dienen und somit zur Reinigung insbesondere von Zahnprothesen und/oder Zähnen verwendet werden kann. Besonders bevorzugt wird als Reinigungsfluid ein Medium verwendet, welches unter Normalbedingungen, also bei 25° C und 1013 mbar, flüssig ist. Ein besonders bevorzugtes Reinigungsfluid ist Wasser.

Unter einem nicht-thermischen Plasma wird insbesondere ein Plasma verstanden, bei welchem eine die Verteilung der kinetischen Energie der Elektronen des Plasmas beschreibende Temperatur, die auch als Elektronen-Temperatur bezeichnet wird, nicht identisch und insbesondere sehr viel höher ist als eine die Verteilung der kinetischen Energie der von dem Plasma umfassten Ionen, insbesondere Atom-Ionen oder Molekül-Ionen, beschreibende Temperatur, die auch als Ionen-Temperatur bezeichnet wird. Dabei ist die Elektronen-Temperatur sehr viel höher als die Ionen-Temperatur, wobei die Ionen-Temperatur insbesondere im Bereich von 25° C bis höchstens 100° C gewählt werden kann. Ein solches Plasma wird aufgrund der vergleichsweise niedrigen Ionen-Temperatur auch als kaltes Plasma bezeichnet.

Besonders bevorzugt ist die Plasmaquelle eingerichtet zur Erzeugung eines solchen nicht-thermischen oder kalten Plasmas bei Atmosphärendruck, insbesondere also bei ungefähr 1013 mbar, wobei ein solches Plasma auch als atmosphärisches Plasma bezeichnet wird.

Als Plasma wird hier insbesondere ein Materiezustand bezeichnet, bei dem geladene Teilchen mit verschiedennamigen Ladungen in der Gasphase nebeneinander vorliegen, wobei sich über ein bestimmten Volumen gemittelt eine neutrale elektrische Ladung für das betrachtete Volumen ergibt. Das Plasma umfasst außerdem vorzugsweise nicht geladene Atome und/oder Moleküle, die sich in elektronisch, vibratorisch oder rotatorisch angeregten Zuständen befinden, und die auch als angeregte Teilchen bezeichnet werden, und/oder freie Radikale, insgesamt also insbesondere nicht-geladene, reaktive Atome und/oder Moleküle, die auch als reaktive Teilchen oder reaktive Spezies bezeichnet werden.

Unter einem Plasmaerzeugnis wird insbesondere das nicht-thermische Plasma selbst oder wenigstens ein Bestandteil oder Produkt des nicht-thermischen Plasmas verstanden, wobei das Plasmaerzeugnis insbesondere aktive und/oder reaktive Spezies aufweist, die Bestandteil des nicht-thermischen Plasmas sind oder aus dem nicht-thermischen Plasma stammen. Das Plasmaerzeugnis umfasst also nicht notwendigerweise den zuvor definierten Materiezustand, bei dem Teilchen verschiedennamiger Ladung nebeneinander vorliegen. Es ist insbesondere möglich, dass das eigentliche Plasma im engeren Sinne bereits abgeklungen ist, während das Plasmaerzeugnis noch vorliegt. Das Plasmaerzeugnis weist bevorzugt insbesondere ionisierte Komponenten, und/oder angeregte oder aktivierte neutrale Komponenten auf.

Unter "plasmahaltiger Luft" oder "plasmahaltigem Gas" wird hier und im Folgenden insbesondere Luft oder Gas verstanden, welche/welches das Plasmaerzeugnis, nämlich insbesondere aktive und/oder reaktive Spezies aufweist, die Bestandteil des erzeugten Plasmas sind oder aus dem erzeugten Plasma stammen. Es ist nicht notwendigerweise erforderlich, dass die Luft oder das Gas tatsächlich noch ein Plasma im engeren Sinne des zuvor definierten Materiezustands aufweisen. Vielmehr genügt es für die Eigenschaft "plasmahaltig" bevorzugt, dass noch aktive und/oder reaktive Spezies, die in dem Plasma oder gemeinsam mit dem Plasma erzeugt wurden, von der Luft oder dem Gas umfasst sind.

Unter einer Plasmaquelle wird insbesondere eine Einrichtung verstanden, die ausgebildet ist, um ein nicht-thermisches Plasma, insbesondere ein atmosphärisches Plasma, zu erzeugen. Bevorzugt handelt es sich dabei um eine elektrische und/oder elektronische Einrichtung, wobei das Plasma durch eine elektrische Entladung, insbesondere eine dielektrische Barriereentladung, eine Koronaentladung, oder eine andere zur Erzeugung eines nicht-thermischen Plasmas geeignete Entladungsform erzeugt wird. Besonders bevorzugt ist die Plasmaquelle als Oberflächen-Mikroentladungsquelle (surface micro discharge - SMD), wie sie beispielsweise in der internationalen Patentanmeldung mit der Veröffentlichungsnummer WO 2010/094304 A1 beschrieben wird, oder nach dem Prinzip der selbststerilisierenden Oberfläche (self-sterilizing surface - SSS), wie sie beispielsweise in der europäischen Patentanmeldung EP 2 387 907 A1, WO 2011/144344 A2 und in WO 2011/110343 A1 beschrieben wird, ausgebildet. Es ist auch möglich, dass die Plasmaquelle nach dem Prinzip der dielektrischen Barriereentladung ausgebildet ist (dielectric barrier discharge - DBD). Die genannten Anmeldungen und Schriften sind per Verweis bezüglich der Ausgestaltung der Plasmaquelle hier einbezogen. Es ist insbesondere möglich, dass die Plasmaquelle eine erste Elektrode und eine zweite Elektrode aufweist, wobei die erste Elektrode von der zweiten Elektrode durch wenigstens ein Dielektrikum beabstandet ist. Dabei kann wenigstens eine der Elektroden auf einer Oberfläche des Dielektrikums angeordnet und/oder in eine Oberfläche des Dielektrikums eingebettet sein. Auf diese Weise ist es möglich, ein Plasma unmittelbar auf einer Oberfläche des Dielektrikums zu erzeugen. Die Plasmaquelle kann eine flächige, insbesondere planare Elektrodenanordnung aufweisen, insbesondere wenn sie in eine Abdeckeinrichtung integriert ist. Es ist aber auch möglich, dass die Plasmaquelle eine gekrümmte, insbesondere zylindrische, oder allgemeiner eine ein Inneres umgreifende, runde, ovale oder mehreckige Form aufweist, insbesondere wenn sie in einer Mediumleitung zur Durchleitung eines gasförmigen Mediums angeordnet ist. Es ist aber auch möglich, in einer solchen Mediumleitung wenigstens eine planare Plasmaquelle oder eine Mehrzahl planarer Plasmaquellen - insbesondere in Form einer Stapelanordnung - anzuordnen. Auch eine perforierte, von einem gasförmigen Medium durchströmbare Elektrodenanordnung ist möglich.

Die Elektrodenanordnung der Plasmaquelle einschließlich des Dielektrikums kann sehr dünn ausgebildet sein. Insbesondere kann diese eine Dicke von 20 µm bis wenige 100 µm, insbesondere von 20 µm bis höchstens 500 µm, vorzugsweise bis höchstens 300 µm, aufweisen.

Eine derart dünne Elektrodenanordnung ist flexibel und kann ohne weiteres in eine gewünschte und insbesondere für die Vorrichtung günstige Form gebracht werden.

Unter einer Durchmischung des Plasmaerzeugnisses mit dem Reinigungsfluid wird insbesondere verstanden, dass das Plasma und/oder insbesondere die von dem Plasma umfassten oder aus dem Plasma stammenden reaktiven Spezies derart mit dem Reinigungsfluid in Kontakt gebracht werden, dass sie mit diesem reagieren und/oder in dem Reinigungsfluid absorbiert werden. Das Durchmischen kann beispielsweise durch Schütteln, Zerstäuben des Reinigungsfluids in einen Bereich, in welchem das Plasmaerzeugnis angeordnet ist, und/oder durch Belüftung des Reinigungsfluids mit plasmahaltigem Gas, insbesondere plasmahaltiger Luft, insbesondere also durch Zufuhr von plasmahaltigem Gas oder Luft in das Reinigungsfluid, erfolgen.

Unter einem aktivierten Reinigungsfluid wird insbesondere das Reinigungsfluid nach Durchmischung mit dem Plasmaerzeugnis verstanden, wobei das Reinigungsfluid Spezies aus dem Plasma, Reaktionsprodukte mit dem Plasmaerzeugnis und/oder solvatisierte Spezies oder Reaktionsprodukte des Plasmaerzeugnisses aufweist. Solche Spezies verleihen dem Reinigungsfluid insbesondere seine reinigende, insbesondere desinfizierende, und/oder bleichende Wirkung.

Unter einer Anwendung des aktivierten Reinigungsfluids auf wenigstens einen Gegenstand, insbesondere auf wenigstens eine Zahnprothese wird insbesondere eine Applikation des aktivierten Reinigungsfluids zum Zwecke der Reinigung, insbesondere Desinfektion, und/oder zum Bleichen auf den wenigstens einen Gegenstand, insbesondere auf wenigstens eine Zahnprothese verstanden. Die Anwendung kann dabei außerhalb des Mundes, beispielsweise in dem Aufnahmevolumen selbst, erfolgen.

Gemäß der Erfindung ist vorgesehen, dass die Plasmaquelle angeordnet ist, um auf einen über einem Füllstand des Reinigungsfluids in dem Aufnahmevolumen angeordneten Luftraum zu wirken. In diesem Fall ist bei der Vorrichtung über einem Füllstand des Reinigungsfluids - für den beispielsweise eine Füllstandsmarkierung vorgesehen sein kann - ein Luftraum angeordnet, wobei die Plasmaquelle so relativ zu dem Aufnahmevolumen und dem bestimmungsgemäß vorgesehenen Luftraum angeordnet ist, dass mittels der Plasmaquelle in dem Luftraum ein Plasma erzeugt werden kann. Das Plasma wird also bevorzugt nicht unmittelbar in dem Reinigungsfluid, sondern vielmehr in dem Luftraum über dem Reinigungsfluid erzeugt. Es ist insbesondere möglich, dass die Plasmaquelle an einer dem Aufnahmevolumen zugeordneten Abdeckeinrichtung angeordnet ist. Insbesondere ist es möglich, dass die Plasmaquelle in die Abdeckeinrichtung integriert ist. Die Abdeckeinrichtung ist vorzugsweise eingerichtet, um das Aufnahmevolumen derart abzudecken, dass ein geschlossenes Volumen ausgebildet wird. Es ist insbesondere möglich, dass die Abdeckeinrichtung als Deckel ausgebildet ist.

Die Abdeckeinrichtung weist vorzugsweise eine Heizeinrichtung auf, mittels der die Abdeckeinrichtung zumindest bereichsweise, insbesondere an einer im Betrieb der Vorrichtung bestimmungsgemäß dem Reinigungsfluid zugewandten Fläche, beheizbar ist, um eine unerwünschte Kondensation an der Abdeckeinrichtung zu verhindern. Die Heizeinrichtung ist bevorzugt als elektrische Heizeinrichtung, insbesondere als Heizdraht oder Heizwicklung, als Infrarotheizung, als thermoelektrisches Element, insbesondere als Peltier- oder Seebeck-Element, oder in anderer geeigneter Weise ausgebildet. Die Plasmaquelle und die Heizeinrichtung können gemeinsam und bevorzugt integral miteinander an der Abdeckeinrichtung vorgesehen sein. Es ist möglich, dass ein Teil, insbesondere eine Elektrode, der Plasmaquelle als Heizelement, insbesondere als Heizdraht, für die Heizeinrichtung verwendet wird.

Es ist möglich, dass ein Durchmischen des Reinigungsfluids mit dem Plasmaerzeugnis zusätzlich durch Schütteln erfolgt, wobei insbesondere nach Erzeugen des Plasmas durch die Plasmaquelle das Aufnahmevolumen mit einer aufgesetzten Abdeckeinrichtung geschüttelt wird, sodass eine Durchmischung des plasmahaltigen Luftraums und des Reinigungsfluids in dem Aufnahmevolumen stattfindet. Es ist dabei möglich, dass zum Schütteln die Abdeckeinrichtung, insbesondere der Deckel, verwendet wird, welche die Plasmaquelle aufweist. Es ist aber auch möglich, dass die Vorrichtung zusätzlich zu einer ersten Abdeckeinrichtung, welche die Plasmaquelle aufweist, eine zweite Abdeckeinrichtung, insbesondere einen zweiten Deckel, aufweist, wobei die zweite Abdeckeinrichtung frei ist von einer Plasmaquelle. Diese kann zum Schütteln verwendet werden. In diesem Fall wird bevorzugt zunächst mit der ersten Abdeckeinrichtung das Plasma erzeugt; anschließend wird die erste Abdeckeinrichtung von dem Aufnahmevolumen entfernt, und die zweite Abdeckeinrichtung wird auf das Abdeckvolumen aufgesetzt, wonach das Abdeckvolumen geschüttelt wird.

Gemäß einer Weiterbildung der Erfindung ist vorgesehen, dass das Aufnahmevolumen in einem Aufnahmegefäß ausgebildet oder angeordnet ist. Es ist auch möglich, dass das Aufnahmevolumen als Aufnahmegefäß ausgebildet ist. Besonders bevorzugt wird ein becherartiges Aufnahmegefäß, insbesondere ein Becher. Dieser kann beispielsweise die Größe und/oder Form eines Zahnbechers oder eines Reinigungsglases für Zahnprothesen haben. Das Aufnahmevolumen kann so in sehr einfacher und zugleich handlicher Art bereitgestellt werden. Bevorzugt ist das Aufnahmegefäß aus der Vorrichtung entnehmbar oder von weiteren Teilen der Vorrichtung trennbar. Das Aufnahmegefäß ist bevorzugt Teil der Vorrichtung.

Gemäß einer Weiterbildung der Erfindung ist vorgesehen, dass die Plasmaquelle separat von dem Aufnahmevolumen angeordnet ist. Dies bedeutet insbesondere, dass die Plasmaquelle von dem Aufnahmevolumen räumlich getrennt, insbesondere außerhalb des Aufnahmegefäßes, welches das Aufnahmevolumen aufweist, angeordnet ist. Es ist gleichwohl möglich, dass die Plasmaquelle derart angeordnet und eingerichtet ist, um ein Plasma in Luft zu erzeugen, welche aus dem Luftraum oberhalb des Reinigungsfluids stammt. Es ist aber auch möglich, dass die Plasmaquelle angeordnet und eingerichtet ist, um ein Plasma in einem anderen Medium, insbesondere in einem anderen gasförmigen Medium zu erzeugen, welches dann bevorzugt anschließend dem Reinigungsfluid in dem Aufnahmevolumen zugeführt wird.

Gemäß einer Weiterbildung der Erfindung ist vorgesehen, dass die Plasmaquelle in einer Mediumleitung zur Durchleitung eines gasförmigen Mediums angeordnet ist. Dabei ist die Plasmaquelle bevorzugt separat von dem Aufnahmevolumen angeordnet. Die Plasmaquelle ist eingerichtet, um ein Plasma in dem durch die Mediumleitung geführten, gasförmigen Medium zu erzeugen. Das Plasma wird dabei bevorzugt außerhalb des Aufnahmevolumens und insbesondere außerhalb des Aufnahmegefäßes erzeugt. Die Mediumleitung weist vorzugsweise ein Medienauslass auf, der in das Aufnahmevolumen für das Reinigungsfluid mündet. Dabei ist der Medienauslass bevorzugt derart angeordnet, dass er unterhalb des bestimmungsgemäßen Füllstands für das Reinigungsfluid in dasselbe mündet, sodass er also mit Bezug auf das Reinigungsfluid submers vorgesehen ist. Das gasförmige Medium, welches das Plasma oder Plasmaerzeugnis aufweist, kann so effizient in das Reinigungsfluid eingeleitet werden.

Gemäß einer Weiterbildung der Erfindung ist vorgesehen, dass die Mediumleitung einen Medieneinlass aufweist, der in den Luftraum über dem bestimmungsgemäßen Füllstand des Reinigungsfluids in dem Aufnahmevolumen mündet. Auf diese Weise kann das gasförmige Medium, insbesondere Luft, dem Luftraum über dem Reinigungsfluid entnommen werden, sodass Plasma - separat von dem Aufnahmevolumen - in der dem Luftraum entstammenden Luft erzeugt und anschließend das Plasmaerzeugnis über den Medienauslass dem Reinigungsfluid zugeführt wird.

Die Plasmaquelle und die Mediumleitung sind dabei bevorzugt so eingerichtet, dass das gasförmige Medium im Kreislauf durch die Mediumleitung und die Plasmaquelle führbar ist.

Insbesondere wird dabei gasförmiges Medium dem Luftraum über dem Füllstand des Reinigungsfluids entnommen, durch die Plasmaquelle geleitet, anschließend in mit dem Plasmaerzeugnis angereichertem Zustand dem Reinigungsfluid zugeführt, durch welches es insbesondere in Form von Gasblasen hindurchperlt, wobei es aus dem Reinigungsfluid wiederum in den Luftraum austritt und dort wieder über den Medieneinlass der Mediumleitung zugeführt wird. Vorzugsweise wird dabei nicht das gesamte Plasma oder die gesamten reaktiven und/oder aktiven Spezies von dem Reinigungsfluid aufgenommen, sodass der Luftraum nach einer gewissen Betriebszeit auch aktive und/oder reaktive Spezies aufweist, die wiederum in den Medieneinlass und zu der Plasmaquelle gelangen. Die Plasmaquelle verstärkt so bei jedem Durchlauf eine Konzentration an aktiven und/oder reaktiven Spezies in dem gasförmigen Medium, wobei zugleich eine Verstärkung der aktivierenden Wirkung des gasförmigen Mediums auf das Reinigungsfluid stattfindet. Es wird so ein geschlossener Kreislauf unter Rückführung von aktiven und/oder reaktiven Spezies zu der Plasmaquelle bereitgestellt, wodurch sich sehr schnell ein aktiviertes Reinigungsfluid herstellen lässt.

Die Plasmaquelle ist vorzugsweise in der Mediumleitung stromabwärts des Medieneinlasses und stromaufwärts des Medienauslasses - bezogen auf eine Strömungsrichtung des gasförmigen Mediums in der Mediumleitung - angeordnet. Vorzugsweise ist in der Mediumleitung eine Fördereinrichtung, insbesondere eine Pumpe, zur Förderung des gasförmigen Mediums entlang der Mediumleitung angeordnet. Die Fördereinrichtung ist dabei besonders bevorzugt stromaufwärts der Plasmaquelle angeordnet.

Es ist möglich, dass der Medieneinlass in der Abdeckeinrichtung angeordnet oder in die Abdeckeinrichtung integriert ist. Er kann so in sehr einfacher Weise mit dem Luftraum über dem Reinigungsfluid in Kontakt gebracht werden.

Gemäß einer bevorzugten Ausgestaltung ist vorgesehen, dass der Medienauslass in einen Aerator oder Belüfter mündet. Dieser kann insbesondere als poröser Körper, insbesondere als poröser Keramikkörper, ausgebildet sein. Auf diese Weise kann das plasmaaktivierte oder plasmahaltige, gasförmige Medium in besonders effizienter Weise in das Reinigungsfluid eingeleitet werden.

Die Anordnung der Plasmaquelle separat von dem Aufnahmevolumen hat im Übrigen den Vorteil, dass eine Abkopplung derselben insbesondere von dem Aufnahmegefäß erfolgen kann, sodass dieses in besonders einfacher und komfortabler Weise von dem Rest der Vorrichtung trennbar ist. Die Anordnung einer Plasmaquelle in einer Mediumleitung ist in besonders einfacher Weise skalierbar, wobei sie ohne weiteres an eine gewünschte Menge zu aktivierenden Reinigungsfluids angepasst werden kann.

Gemäß einer Weiterbildung der Erfindung ist vorgesehen, dass die Vorrichtung einen Sockel aufweist, der eine Stellfläche zur Anordnung des Aufnahmegefäßes aufweist. Somit ist ein definierter Bereich für die Anordnung des Aufnahmegefäßes geschaffen.

Bevorzugt ist vorgesehen, dass eine Steuereinrichtung und/oder eine elektrische Speichereinrichtung für die Plasmaquelle in dem Sockel angeordnet ist/sind. Unter einer Steuereinrichtung wird dabei insbesondere eine Einrichtung verstanden, mittels welcher die Plasmaquelle ansteuerbar, insbesondere aktivierbar und deaktivierbar ist. Unter einer elektrischen Speichereinrichtung wird insbesondere eine Einrichtung verstanden, mit welcher elektrische Energie zum Betrieb der Plasmaquelle gespeichert werden kann. Hierbei kann es sich insbesondere um einen Kondensator, eine Batterie oder um einen Akkumulator handeln. Ist die Steuereinrichtung und/oder die elektrische Speichereinrichtung in dem Sockel angeordnet, und ist zugleich die Plasmaquelle in einer Abdeckeinrichtung angeordnet, so sind bevorzugt die Abdeckeinrichtung und der Sockel miteinander elektrisch verbunden. Dies kann beispielsweise über wenigstens eine elektrische Leitung und/oder über einen dem Sockel und der Abdeckeinrichtung gemeinsamen Grundkörper verwirklicht sein.

Es ist möglich, dass der Sockel eine induktive Spannungsversorgung, insbesondere ein Induktionsladegerät, aufweist. In diesem Fall ist vorzugsweise in den Boden des Aufnahmegefäßes eine Induktionsspule, insbesondere eine Qi-Spule, zur Wirkverbindung mit der induktiven Spannungsversorgung in dem Sockel angeordnet.

Alternativ oder zusätzlich ist bevorzugt vorgesehen, dass die Steuereinrichtung und/oder die elektrische Speichereinrichtung in einer dem Aufnahmegefäß zugeordneten Abdeckeinrichtung angeordnet ist/sind. Ist in diesem Fall auch die Plasmaquelle in der Abdeckeinrichtung angeordnet, ergibt sich eine besonders einfache und auch kurze Kontaktierung zwischen der Steuereinrichtung und/oder der elektrischen Speichereinrichtung einerseits und der Plasmaquelle andererseits.

Alternativ oder zusätzlich ist bevorzugt vorgesehen, dass die Steuereinrichtung und/oder die elektrische Speichereinrichtung in einem Griff, insbesondere Handgriff, des Aufnahmegefäßes angeordnet sind. Dabei kann insbesondere die Abdeckeinrichtung in einfacher Weise beispielsweise mit einer im Boden des Aufnahmegefäßes angeordneten Induktionsspule verbunden werden.

Dass die Steuereinrichtung und/oder die elektrische Speichereinrichtung für die Plasmaquelle vorgesehen sind, bedeutet insbesondere, dass die Steuereinrichtung und/oder die elektrische Speichereinrichtung mit der Plasmaquelle wirkverbunden, insbesondere elektrisch wirkverbunden ist/sind, sodass die Plasmaquelle durch die Steuereinrichtung ansteuerbar ist, und/oder so, dass die Plasmaquelle durch die elektrische Speichereinrichtung mit elektrischer Energie versorgbar ist.

Gemäß einer Weiterbildung der Erfindung ist vorgesehen, dass das Aufnahmegefäß eingerichtet ist zur Aufnahme wenigstens einer Zahnprothese. Das Aufnahmegefäß weist dabei insbesondere Abmessungen auf, die zur Aufnahme der wenigstens einen Zahnprothese geeignet sind, jedoch vorzugsweise nicht deutlich über die Abmessungen der wenigstens einen Zahnprothese hinausgehen, wie dies bei einem herkömmlichen Glas oder Becher zur Reinigung von Zahnprothesen oder einem Zahnbecher der Fall ist. Insbesondere weist das Aufnahmegefäß vorzugsweise ein Volumen auf, das höchstens zweimal dem Volumen einer Vollgebiss-Prothese entspricht. Die Vorrichtung wird hierdurch insgesamt handlich und insbesondere ohne weiteres beispielsweise in einem Badezimmer anordenbar.

Alternativ oder zusätzlich ist bevorzugt vorgesehen, dass die Vorrichtung eine Applikationseinrichtung zur Zuführung des aktivierten Reinigungsfluids in den Mund eines Anwenders aufweist. Dabei ist das Reinigungsfluid bevorzugt in dem Aufnahmevolumen aktivierbar, insbesondere also mit dem Plasmaerzeugnis durchmischbar, und anschließend mittels der Applikationseinrichtung von dem Aufnahmevolumen in den Mund des Anwenders führbar. Besonders bevorzugt ist die Vorrichtung als Munddusche ausgebildet, wobei sie bevorzugt eine Munddüse aufweist, welcher das aktivierte Reinigungsfluid aus dem Aufnahmevolumen zuführbar ist, und durch welche das aktivierte Reinigungsfluid in den Mund eines Anwenders einbringbar ist.

Gemäß einer Weiterbildung der Erfindung ist vorgesehen, dass die Vorrichtung einen Anordnungsbereich zur Anordnung des Aufnahmegefäßes aufweist. Dies kann beispielsweise eine Stellfläche auf einem Sockel, aber auch ein andersartiger Anordnungsbereich sein. Vorzugsweise weist die Vorrichtung eine Abdeckeinrichtung derart an einem Grundkörper der Vorrichtung auf, dass das Aufnahmegefäß durch die Abdeckeinrichtung abdeckbar, vorzugsweise abgedeckt ist, wenn es in dem Anordnungsbereich angeordnet ist. Beispielsweise kann das Aufnahmegefäß zum Abdecken mit der Abdeckeinrichtung unter die Abdeckeinrichtung geschoben werden, wodurch es in dem Anordnungsbereich angeordnet wird.

Besonders bevorzugt ist die Abdeckeinrichtung derart verlagerbar an dem Grundkörper der Vorrichtung angeordnet, dass sie in eine das Aufnahmegefäß abdeckende Abdeckposition verlagert wird, wenn das Aufnahmegefäß in dem Anordnungsbereich angeordnet wird. Es ist beispielsweise möglich, dass die Abdeckeinrichtung um wenigstens eine Achse schwenkbar gelagert ist, wobei eine Verlagerungseinrichtung derart vorgesehen sein kann, dass die Abdeckeinrichtung automatisch in die Abdeckposition verlagert wird, wenn das Aufnahmegefäß in den Anordnungsbereich verlagert wird. Bevorzugt wird die Abdeckeinrichtung in eine Öffnungsposition verlagert, wenn das Aufnahmegefäß aus dem Anordnungsbereich entfernt wird. Dies entspricht beispielsweise der Funktionsweise bestimmter Kaffeemaschinen, bei denen eine Brühgruppe automatisch und/oder zwangsgesteuert auf einer Kaffeekanne angeordnet wird, wenn die Kaffeekanne in einen bestimmten Brühbereich der Kaffeemaschine verlagert wird. Es ist aber alternativ auch möglich, dass die Abdeckeinrichtung manuell in die Abdeckposition einerseits und in die Öffnungsposition andererseits verlagerbar ist, ohne Zwangssteuerung durch das Aufnahmegefäß.

Bei einem besonders bevorzugten Ausführungsbeispiel weist die Vorrichtung eine automatische Aktivierungseinrichtung für die Plasmaquelle auf, wobei diese bevorzugt automatisch aktiviert wird, wenn das Aufnahmegefäß in dem Anordnungsbereich angeordnet wird. Dies kann insbesondere kombiniert werden mit einer automatisch bei Anordnung des Aufnahmegefäßes in dem Anordnungsbereich in ihre Abdeckposition verlagerbaren Abdeckeinrichtung. Ist die Plasmaquelle in einer Mediumleitung zur Durchleitung eines gasförmigen Mediums angeordnet, und weist die Mediumleitung eine Fördereinrichtung zur Förderung des gasförmigen Mediums entlang der Mediumleitung auf, kann zusätzlich oder alternativ auch diese Fördereinrichtung automatisch bei Anordnung des Aufnahmegefäßes in dem Anordnungsbereich aktivierbar sein.

Gemäß der Erfindung ist vorgesehen, dass die Vorrichtung eine Mischeinrichtung zur Durchmischung des Plasmaerzeugnisses mit dem Reinigungsfluid aufweist. Auf diese Weise kann insbesondere ein vergleichsweise mühseliges Schütteln des Aufnahmevolumens vermieden werden, weil eine Durchmischung des Plasmaerzeugnisses mit dem Reinigungsfluid vielmehr mit der Mischeinrichtung erfolgen kann.

Gemäß einer nicht zur Erfindung gehörenden Ausgestaltung der Vorrichtung ist es möglich, dass die Mischeinrichtung einen Zerstäuber aufweist. Der Zerstäuber ist bevorzugt eingerichtet, um das Reinigungsfluid - insbesondere in einen Bereich, in dem das Plasmaerzeugnis vorliegt, insbesondere in einen Luftraum über dem Aufnahmevolumen - hinein zu zerstäuben. Der Zerstäuber kann insbesondere als Piezo-Zerstäuber, als Ultraschall-Zerstäuber, oder in anderer geeigneter Weise ausgebildet sein.

Zusätzlich oder alternativ ist es möglich, dass die Mischeinrichtung einen Aerator oder Belüfter aufweist. Darunter wird eine Vorrichtung verstanden, die eingerichtet ist, um plasmahaltiges Gas oder plasmahaltige Luft in das Reinigungsfluid einzuleiten. Dabei kann es sich insbesondere um einen porösen Körper handeln, der in dem Reinigungsfluid anordenbar ist, wobei durch den porösen Körper plasmahaltiges Gas oder plasmahaltige Luft durchführbar und aus einer Vielzahl von in dem porösen Körper ausgebildeten Kanälen in das Reinigungsfluid einbringbar ist. Insbesondere kann der poröse Körper als Keramikkörper ausgebildet sein.

Insbesondere über einen Belüfter oder Aerator kann dem Reinigungsfluid auch ein extern und/oder separat von der restlichen Vorrichtung erzeugtes Plasmaerzeugnis zugeführt werden.

Zusätzlich oder alternativ ist bevorzugt vorgesehen, dass die Mischeinrichtung einen Medienauslass einer die Plasmaquelle aufweisenden Mediumleitung aufweist, wobei der Medienauslass bevorzugt in das Aufnahmevolumen für das Reinigungsfluid mündet und insbesondere mit Bezug auf einen bestimmungsgemäßen Füllstand des Reinigungsfluids submers vorgesehen ist. Dabei ist es möglich, dass der Medienauslass in einen Aerator oder Belüfter mündet.

Die Mischeinrichtung ist erfindungsgemäß in dem Aufnahmevolumen, insbesondere in dem Aufnahmegefäß, angeordnet. Das Aufnahmegefäß weist vorzugsweise wenigstens eine Leistungs- und/oder Mediendurchführung auf, durch welche es möglich ist, der Mischeinrichtung durch eine Wandung - insbesondere einen Boden - des Aufnahmegefäßes hindurch Leistung, insbesondere elektrische Leistung, und/oder ein Medium, insbesondere plasmahaltiges Gas oder plasmahaltige Luft, zuzuführen. Ein Anschluss für die Durchführung kann beispielsweise in einen Sockel der Vorrichtung integriert sein, welcher eine Stellfläche für das Aufnahmegefäß aufweist. Es ist erfindungsgemäß vorgesehen, dass die Vorrichtung eingerichtet ist, um einem Luftvolumen über einem Füllstand des Reinigungsfluids - gegebenenfalls plasmahaltige(s) - Gas oder Luft zu entnehmen und diese der Mischeinrichtung in dem Aufnahmevolumen - unterhalb des Füllstands - zuzuführen.

Eine hierfür ausgebildete Fluidleitung kann bevorzugt in einem Griff, insbesondere Handgriff, des Aufnahmegefäßes angeordnet sein. Die Fluidleitung kann eine Fördereinrichtung, insbesondere eine Membranpumpe, aufweisen.

Gemäß einer Weiterbildung der Erfindung ist vorgesehen, dass die Vorrichtung eine Gasabtrenneinrichtung aufweist, die eingerichtet ist, um Gas aus dem Luftraum über dem Reinigungsfluid von dem aktivierten Reinigungsfluid abzutrennen. Mittels der Gasabtrenneinrichtung ist es möglich, das aktivierte Reinigungsfluid zu entnehmen und insbesondere im Mund eines Anwenders anzuwenden, ohne zugleich gasförmige Reste des Plasmaerzeugnisses, insbesondere Ozon, in die Nähe der Atmungsorgane des Anwenders zu bringen. Dies ist besonders vorteilhaft bei einer als Munddusche ausgebildeten Vorrichtung.

Vorzugsweise ist die Gasabtrenneinrichtung dadurch gebildet, dass eine Entnahmestelle für das aktivierte Reinigungsfluid unterhalb des bestimmungsgemäßen oder vorbestimmten Füllstands des Reinigungsfluids angeordnet ist, also submers.

Die Gasabtrenneinrichtung kann in einfacher Weise als Tülle oder Schnabelausguß an dem Aufnahmegefäß ausgebildet sein, die/der unterhalb des bestimmungsgemäßen Füllstands, insbesondere in Bodennähe des Aufnahmegefäßes, aus dem Aufnahmevolumen ausmündet. Das aktivierte Reinigungsfluid ist dann in einfacher Weise ausgießbar. Die Abdeckeinrichtung kann danach noch eine gewisse Zeit geschlossen gehalten werden, bis das Plasmaerzeugnis vollständig oder zumindest soweit abgebaut ist, dass eine Gesundheitsgefährdung durch Inhalation ausgeschlossen ist.

Es ist möglich, dass in der Tülle oder dem Schnabelausguß - insbesondere in Höhe des bestimmungsgemäßen Füllstands oder darüber - ein Kugel- oder Ballventil angeordnet ist, um das Aufnahmevolumen nach außen abzuschließen, wenn kein aktiviertes Reinigungsfluid entnommen wird. Hierdurch kann insbesondere ein Entweichen plasmahaltiger Luft- oder Gasbläschen aus dem aktivierten Reinigungsfluid verhindert werden.

Die Gasabtrenneinrichtung kann auch als submers angeordnete Entnahmestelle ausgebildet sein, die über eine Fluidleitung mit einer Munddüse verbunden ist. In einer solchen Fluidleitung kann ein Rückschlagventil, insbesondere eine Ball- oder Kugelventil, angeordnet sein.

Die Aufgabe wird auch gelöst, indem ein Verfahren zur Behandlung, insbesondere zur Reinigung, von Gegenständen, insbesondere von Zahnprothesen geschaffen wird, welches folgende Schritte aufweist: Es wird ein nicht-thermisches Plasma erzeugt; ein Plasmaerzeugnis des nicht-thermischen Plasmas wird mit einem Reinigungsfluid gemischt; das Reinigungsfluid wird durch das Plasmaerzeugnis aktiviert, und das aktivierte Reinigungsfluid wird auf einen Gegenstand, insbesondere auf wenigstens eine Zahnprothese außerhalb des Mundes eines Anwenders, angewendet. In Zusammenhang mit dem Verfahren verwirklichen sich insbesondere die Vorteile, die bereits in Zusammenhang mit der Vorrichtung erläutert wurden. Insbesondere wird im Rahmen des Verfahrens vorzugsweise eine Vorrichtung nach einem der zuvor beschriebenen Ausführungsbeispiele verwendet. Die Vorrichtung ist analog bevorzugt eingerichtet zur Durchführung von einer der im Folgenden beschriebenen Ausführungsformen des Verfahrens.

Vorzugsweise werden im Rahmen des Verfahrens wenigstens eine Zahnprothese desinfiziert, insbesondere wird eine Bakterien- und/oder Virenbeladung auf der wenigstens einen Zahnprothese reduziert. Alternativ oder zusätzlich ist es möglich, dass die wenigstens eine Zahnprothese gebleicht wird/werden.

Das Plasma wird bevorzugt in einem Luftraum oberhalb eines Füllstandes des Reinigungsfluids, welchen dieses in einem Aufnahmevolumen aufweist, oder separat von dem Aufnahmevolumen erzeugt.

Das Mischen erfolgt vorzugsweise derart, dass Plasmabestandteile, insbesondere reaktive Spezies aus dem Plasma, von dem Reinigungsfluid absorbiert werden und/oder mit dem Reinigungsfluid reagieren. Es ist möglich, dass dabei neue oder weitere aktive Spezies erzeugt werden, die letztlich bei der Behandlung von wenigstens einer Zahnprothese wirksam sind.

Das aktivierte Reinigungsfluid wird erfindungsgemäß auf die wenigstens eine Zahnprothese außerhalb des Mundes eines Anwenders, insbesondere in dem Aufnahmevolumen, angewendet.

Das Mischen kann insbesondere durch Schütteln, Zerstäuben des Reinigungsfluids, und/oder durch Belüften oder Aerifizieren des Reinigungsfluids mit plasmahaltiger Luft oder einem plasmahaltigen Gas durchgeführt werden.

Es ist möglich, dass das Aktivieren des Reinigungsfluids durch das Plasmaerzeugnis, insbesondere das Mischen des Plasmaerzeugnisses mit dem Reinigungsfluid, zeitgleich mit dem Anwenden des Reinigungsfluids auf den zu behandelnden Gegenstand, insbesondere die Zahnprothese, durchgeführt wird. Es ist auch möglich, dass zuerst das Reinigungsfluid aktiviert wird, wobei dann das aktivierte Reinigungsfluid zu einem späteren Zeitpunkt auf den zu behandelnden Gegenstand angewendet wird. Vor und/oder während der Behandlung wird vorzugsweise das Reinigungsfluid fortlaufend gerührt oder umgewälzt, und/oder es wird fortlaufend eine Vermischung von plasmahaltiger Luft oder einem plasmahaltigen Gas mit dem Reinigungsfluid durchgeführt.

Gemäß einer Weiterbildung der Erfindung ist vorgesehen, dass ein Verhältnis von einem Luftvolumen, in welchem das nicht-thermische Plasma erzeugt wird, zu einem Volumen des Reinigungsfluids so gewählt wird, dass es wenigstens 1:3 bis höchstens 3:1 beträgt. Zusätzlich oder alternativ ist es auch möglich, dass ein Verhältnis von einer Luftmasse, in welcher das nicht-thermische Plasma erzeugt wird, zu einer Masse des Reinigungsfluids so gewählt wird, dass es wenigstens 1:3 bis höchstens 3:1 beträgt. Mit dem Begriff "Luft" ist hier allgemein ein Trägergas angesprochen, in welchem das nicht-thermische Plasma erzeugt wird. Es kann sich dabei um herkömmliche Umgebungsluft, jedoch auch um ein beliebiges anderes Gas, insbesondere auch um ein Rein- oder Reinstgas, oder um ein Gasgemisch handeln. In einem besonders einfachen Fall des Verfahrens wird allerdings Umgebungsluft zur Erzeugung des nicht-thermischen Plasmas verwendet. Es bedarf dann keiner zusätzlichen Bereitstellung eines Gases. Das hier spezifizierte Verhältnis der Volumina und/oder Massen von Luft zu Reinigungsfluid stellt eine äußerst effiziente Aktivierung des Reinigungsfluids bei zugleich optimalen Behandlungseigenschaften des aktivierten Reinigungsfluids sicher. Es hat sich nämlich herausgestellt, dass dieses Verhältnis wichtig ist insbesondere für die bakterizide und/oder viruzide Wirkung des aktivierten Reinigungsfluids.

Gemäß einer Weiterbildung der Erfindung ist vorgesehen, dass eine Einschaltdauer einer Plasmaquelle für die Erzeugung des nicht-thermischen Plasmas so gewählt wird, dass aktive Sauerstoff-Spezies oder aktive Stickstoff-Spezies in dem nicht-thermischen Plasma und/oder dem Plasmaerzeugnis - insbesondere bezüglich ihrer Konzentration - überwiegen. Unter einer Einschaltdauer ist dabei insbesondere eine integrale Gesamtaktivierungsdauer der Plasmaquelle während eines Plasmaerzeugungsereignisses angesprochen, die sich von einer tatsächlichen Aktivierungsdauer der Plasmaquelle dann nicht unterscheidet, wenn die Plasmaquelle kontinuierlich betrieben wird. Wird die Plasmaquelle allerdings diskontinuierlich, insbesondere gepulst betrieben, sind einzelne, zeitlich voneinander separierte Aktivierungsphasen der Plasmaquelle kleiner als die gesamte Einschaltdauer während des Plasmaerzeugungsereignisses. Die Gesamteinschaltdauer oder integrale Einschaltdauer ist dann insbesondere die Summe der Aktivierungsphasen der Plasmaquelle einschließlich der zwischen diesen Ereignissen angeordneten inaktiven Phasen.

Alternativ oder zusätzlich ist es möglich, dass eine Leistung der Plasmaquelle für die Erzeugung des nicht-thermischen Plasmas so gewählt wird, dass aktive Sauerstoff-Spezies oder aktive Stickstoff-Spezies in dem nicht-thermischen Plasma und/oder dem Plasmaerzeugnis überwiegen. Über die Einschaltdauer und/oder die Leistung der Plasmaquelle kann also in vorteilhafter Weise eingestellt werden, ob in dem nicht-thermischen Plasma und/oder dem Plasmaerzeugnis aktive Sauerstoff-Spezies oder aktive Stickstoff-Spezies überwiegen. Dadurch lässt sich insbesondere die Wirkungsweise des nicht-thermischen Plasmas und/oder des Plasmaerzeugnisses und damit letztlich auch des aktivierten Reinigungsfluids beeinflussen.

Unter aktiven Sauerstoffs-Spezies werden insbesondere Moleküle, Ionen, insbesondere Molekül-Ionen, Radikale, angeregte Atome oder Moleküle, oder auch besonders reaktive Moleküle bezeichnet, die im Wesentlichen aus Sauerstoff gebildet sind und/oder im Wesentlichen Sauerstoffatome aufweisen. Ein Beispiel für eine solche aktive Sauerstoff-Spezies ist Ozon.

Unter aktiven Stickstoff-Spezies werden eben solche Spezies bezeichnet, die aus Stickstoff gebildet sind und/oder Stickstoff-Atome aufweisen. Beispiele für aktive Stickstoff-Spezies sind insbesondere Stickoxide.

Eine Betrachtung der Reaktionskinetiken der Sauerstoffchemie einerseits und der Stickstoffchemie andererseits im Plasma zeigt, dass Geschwindigkeitskonstanten der Ozonbildung, insbesondere diejenige eines geschwindigkeitsbestimmenden Schritts, schneller sind als Geschwindigkeitskonstanten für die Bildung von Stickoxiden, und insbesondere schneller als eine Geschwindigkeitskonstante für die Bildung von Stickstoffdioxid aus Stickstoff und Sauerstoff ohne Beteiligung von Ozon als Zwischenprodukt. Ozon wird also insbesondere auf einer schnelleren Zeitskala gebildet, als Stickoxide ohne Beteiligung von Ozon. Es existieren allerdings auch Reaktionsmechanismen, bei denen Stickoxide unter Beteiligung von Ozon gebildet werden. Diese Reaktionsschritte setzen selbstverständlich die Anwesenheit von Ozon voraus, sodass die Bildung von Stickoxiden über solche Reaktionsmechanismen der Ozonbildung nachgelagert ist. Insbesondere wird Ozon auch durch bereits gebildete, sauerstoffärmere Stickoxide abgebaut, wobei diese Stickoxide zu sauerstoffreicheren Stickoxiden aufoxidiert werden.

Insgesamt ergibt sich so, dass zu kürzeren Einschaltdauern und kleineren Leistungen der Plasmaquelle hin die Sauerstoffchemie überwiegt, während zu längeren Einschaltdauern und höheren Leistungen der Plasmaquelle hin die Stickstoffchemie überwiegt.

Die jeweils für die Plasmachemie relevanten, konkreten Parameter mit Bezug auf die Einschaltdauer und/oder die Leistung der Plasmaquelle müssen für eine konkrete Konfiguration der Vorrichtung und insbesondere auch der Plasmaquelle durch Berechnungen und/oder Versuche ermittelt werden. Dies ist allerdings ohne weiteres möglich. Beispielsweise liegt eine Ozonerzeugungsrate für eine Oberflächenmikroentladungs-Plasmaquelle in Luft bei normalen atmosphärischen Bedingungen im Bereich von 50 ppm/cm²s bis 200 ppm/cm²s. Hieraus lässt sich dann für ein vorgegebenes Luftvolumen die Behandlungszeit ausrechnen, um eine bestimmte Plasmachemie bereitzustellen.

Es hat sich gezeigt, dass aktive Stickstoff-Spezies insbesondere relevant sind für eine gute Langzeitwirkung des aktivierten Reinigungsfluids als Bakterizid. Demgegenüber ist mit Blick auf die Trinkwasserqualität des Reinigungsfluids ein Überwiegen aktiver Sauerstoff-Spezies besser geeignet.

Gemäß einer Weiterbildung der Erfindung ist vorgesehen, dass als Reinigungsfluid Wasser verwendet wird. Dabei gestaltet sich insbesondere die Behandlung der wenigstens einen Zahnprothese besonders einfach, da Wasser typischerweise ohne weiteres zur Verfügung steht. Besonders bevorzugt wird Trinkwasser, ganz bevorzugt Leitungswasser verwendet, das typischerweise in einfacher und kostengünstiger Weise sowie ausreichender Menge zur Verfügung steht. Weiterhin weist Leitungswasser typischerweise auch Trinkwasserqualität auf. Vorteilhaft bei der vorgeschlagenen Vorrichtung und dem hier vorgeschlagenen Verfahren ist, dass auch das aktivierte Reinigungsfluid bevorzugt Trinkwasserqualität aufweist, sodass diese Eigenschaft des als Reinigungsfluids verwendeten Wassers nicht durch die Aktivierung mit dem Plasmaerzeugnis verloren geht. Es ist in nicht zur Erfindung gehörender Weise auch möglich, dass das aktivierte Reinigungsfluid also ohne weiteres in den Mund eines Patienten eingebracht und auch geschluckt werden kann, ohne dass deswegen gesundheitliche Bedenken zu befürchten sind.

Alternativ ist es möglich, dass als Reinigungsfluid eine alkoholische Lösung verwendet wird. Unter einer alkoholischen Lösung wird insbesondere eine Lösung verstanden, die wenigstens einen Alkohol, insbesondere Ethanol oder einen anderen Alkohol, als Lösungsmittel und/oder als Gelöstes aufweist. Insbesondere kann als alkoholische Lösung eine Lösung wenigstens eines Alkohols in Wasser, mithin eine wässrige, alkoholische Lösung, oder eine Lösung von Wasser in wenigstens einem Alkohol verwendet werden. Der von der Lösung umfasste Alkohol kann zusätzlich zur Behandlung der wenigstens einen Zahnprothese beitragen. Es ist auch möglich, dass das Plasmaerzeugnis in Kombination mit dem wenigstens einen Alkohol weitere aktive Spezies bildet, insbesondere durch Reaktion mit dem Alkohol.

Es ist auch möglich, dass als Reinigungsfluid sauerstoffangereichertes Wasser verwendet wird. Dabei kann insbesondere die Sauerstoffchemie beim Aktivieren des Reinigungsfluids verstärkt werden, sodass vermehrt aktive Sauerstoff-Spezies, insbesondere Ozon, aber auch Wasserstoffperoxid, gebildet werden. Um das Wasser mit Sauerstoff anzureichern, können beispielsweise Sauerstofftabletten verwendet werden. Solche Sauerstofftabletten weisen typischerweise sprudelnde Eigenschaften auf, wodurch sie auch die Absorption von wirksamen Spezies des nicht-thermischen Plasmas im Wasser beschleunigen könnten, indem sie nämlich zur Bildung feiner Wassertröpfchen beitragen. Weiterhin könnten solche Sauerstofftabletten auch die Luftchemie in dem Luftraum, in welchem das nicht-thermische Plasma gebildet wird, beeinflussen, sodass hier die Art und Anzahl der entstehenden aktiven Spezies verändert wird.

Es ist auch möglich, dass als Reinigungsfluid eine phosphatgepufferte Salzlösung verwendet wird. Eine solche Lösung ermöglicht das Arbeiten bei einem konstanten pH-Wert. Durch verschiedene Salze besitzt die Lösung den osmotischen Druck des menschlichen Organismus und ist demanch eine isotonische Salzlösung. Eine solche Lösung enthält vorzugsweise 137 mM Natriumchlorid, 2,7 mM Kaliumchlorid und 12 mM Gesamt-Phosphat, insbesondere in Form von HPO₄²⁻ und H₂PO₄⁻. Der pH-Wert einer solchen phosphatgepufferten Salzlösung ist vorzugsweise 7,4. Ein Liter einer solchen Lösung enthält vorzugsweise 8,0 g Natriumchlorid, 0,2 g Kaliumchlorid, 1,42 g Dinatriumhydrogenphosphat oder 1,78 Dinatriumhydrogenphosphat-Dihydrat, und 0,27 g Kaliumdihydrogenphosphat. Eine phosphatgepufferte, isotonische Salzlösung ist physiologisch besonders unbedenklich.

Gemäß einer Weiterbildung der Erfindung ist vorgesehen, dass ein aktiviertes Reinigungsfluid erzeugt wird, welches einen Wasserstoffperoxid-Gehalt aufweist, der kleiner ist als der in der Europäischen Union an dem den Zeitrang der vorliegenden Anmeldung bestimmenden Tag geltende Grenzwert für nicht-professionelles Zahnbleichen (derzeit 0,1 %). Der Gehalt an Wasserstoffperoxid ist insbesondere bevorzugt kleiner als 0,1 % - vorzugsweise hier und im Folgenden angegeben in Vol.-%. Herkömmliche Bleichmittel, die zum professionellen Bleichen von Zahnprothesen und/oder Zähnen verwendet werden, weisen sehr viel höhere Wasserstoffperoxid-Gehalte auf (bis zu 6 %), die insbesondere ungefähr zehnmal höher sein können als der gesetzlich vorgegebene Grenzwert für nicht-professionelles Zahnbleichen (derzeit 0,1 %). Es hat sich gezeigt, dass das aktivierte Reinigungsfluid gemäß der hier vorgeschlagenen Vorrichtung und dem hier vorgeschlagenen Verfahren aufgrund weiterer aktiver und insbesondere bleichwirksamer Spezies eine gleiche Bleichwirkung wie bei einem Wasserstoffperoxid-Gehalt von 1 % und trotzdem einen deutlich niedrigeren Wasserstoffperoxid-Gehalt insbesondere von weniger als 0,1 % aufweisen kann.

Alternativ oder zusätzlich wird bevorzugt ein aktiviertes Reinigungsfluid erzeugt, welches Trinkwasserqualität aufweist. Unter Trinkwasserqualität wird dabei verstanden, dass das aktivierte Reinigungsfluid wie Trinkwasser ohne negative gesundheitliche Auswirkungen durch den Menschen trinkbar ist. Bevorzugt wird unter Trinkwasserqualität eine Übereinstimmung des aktivierten Reinigungsfluids mit gesetzlichen Trinkwassernormen, insbesondere der gesetzlichen Trinkwassernorm der Europäischen Union, an dem für den Zeitrang der vorliegenden Anmeldung bestimmenden Tag verstanden. Aufgrund der Vielzahl verschiedener reaktiver Spezies in dem aktivierten Reinigungsfluid, die in komplizierten Prozessen zusammenspielen, um eine effiziente Behandlung von Zahnprothesen und/oder Zähnen zu bewirken, können einzelne Konzentrationen dieser Spezies niedrig genug sein, dass das aktivierte Reinigungsfluid Trinkwasserqualität aufweist.

Die Beschreibung der Vorrichtung einerseits und das Verfahren andererseits sind komplementär zueinander zu verstehen. Merkmale der Vorrichtung, die explizit oder implizit in Zusammenhang mit dem Verfahren beschrieben wurden, sind vorzugsweise einzeln oder miteinander kombiniert Merkmale eines bevorzugten Ausführungsbeispiels der Vorrichtung. Verfahrensschritte, die explizit oder implizit in Zusammenhang mit der Vorrichtung beschreiben wurden, sind bevorzugt einzeln oder miteinander kombiniert Schritte einer bevorzugten Ausführungsform des Verfahrens. Das Verfahren zeichnet sich bevorzugt durch wenigstens einen Schritt aus, der durch wenigstens ein Merkmal eines erfindungsgemäßen oder bevorzugten Ausführungsbeispiels der Vorrichtung bedingt ist. Die Vorrichtung zeichnet sich bevorzugt durch wenigstens ein Merkmal aus, welches durch wenigstens einen Schritt einer erfindungsgemäßen oder bevorzugten Ausführungsform des Verfahrens bedingt ist.

Die Erfindung wird im Folgenden anhand der Zeichnung näher erläutert. Dabei zeigen:
- Figur 1: eine schematische Darstellung eines ersten, nicht zur Erfindung gehörenden Beispiels einer Vorrichtung zur Behandlung von Gegenständen, insbesondere von Zahnprothesen und/oder Zähnen;
- Figur 2: eine schematische Detaildarstellung des Beispiels gemäß Figur 1;
- Figur 3: eine schematische Darstellung eines ersten Ausführungsbeispiels der Vorrichtung;
- Figur 4: eine schematische Darstellung eines zweiten Ausführungsbeispiels der Vorrichtung;
- Figur 5: eine schematische Darstellung eines drittenAusführungsbeispiels der Vorrichtung;
- Figur 6: eine Darstellung eines vierten Ausführungsbeispiels der Vorrichtung;
- Figur 7: eine Darstellung eines zweiten, nicht zur Erfindung gehörenden Beispiels der Vorrichtung;
- Figur 8: eine Darstellung eines fünften Ausführungsbeispiels der Vorrichtung;
- Figur 9: eine diagrammatische Darstellung eines Zusammenhangs zwischen einer Durchmischungszeit, der Einschaltdauer einer Plasmaquelle, und der bakteriziden Wirkung eines mittels der erfindungsgemäßen Vorrichtung und eines erfindungsgemäßen Verfahrens hergestellten, aktivierten Reinigungsfluids, und
- Figur 10: eine diagrammatische Darstellung der Effizienz einer Ausführungsform des erfindungsgemäßen Verfahrens zum Bleichen von Gegenständen, insbesondere zum Zahnbleichen.

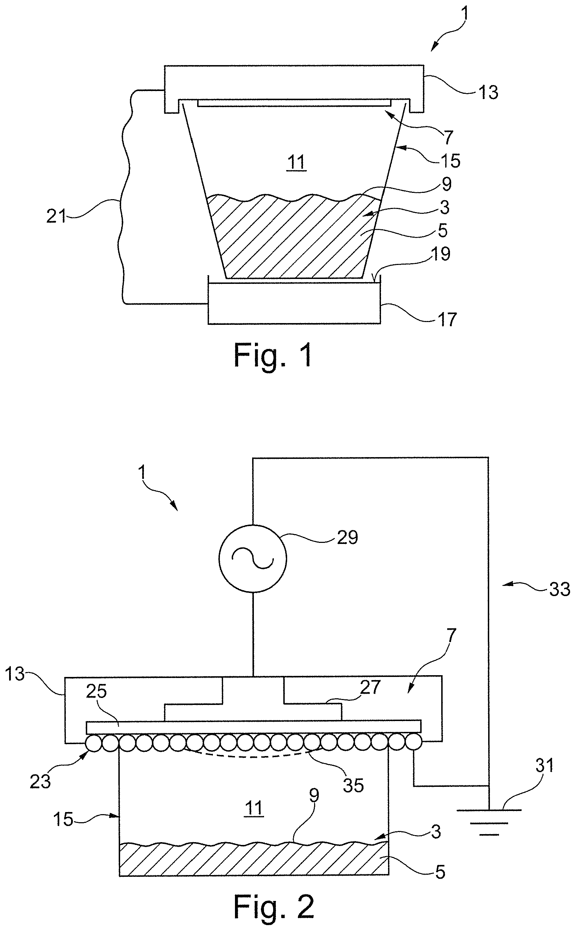

**Fig. 1** zeigt eine schematische Darstellung eines ersten, nicht zur Erfindung gehörenden Beispiels einer Vorrichtung 1 zur Behandlung, insbesondere zur Reinigung, ganz besonders zur Desinfektion und/oder zum Bleichen, von Gegenständen, insbesondere von Zahnprothesen und/oder Zähnen. Die Vorrichtung 1 weist ein Aufnahmevolumen 3 auf, das eingerichtet ist zur Aufnahme eines Reinigungsfluids 5, wobei als Reinigungsfluid 5 bevorzugt Wasser, insbesondere Trink- oder Leitungswasser, eine alkoholische Lösung, mit Sauerstoff angereichertes Wasser, und/oder eine phosphatgepufferte Salzlösung verwendet wird.

Die Vorrichtung 1 weist eine Plasmaquelle 7 auf, die eingerichtet ist zur Erzeugung eines nicht-thermischen Plasmas. Die Plasmaquelle 7 ist bei dem hier dargestellten Ausführungsbeispiel so angeordnet, dass sie auf einen über einem Füllstand 9 des Reinigungsfluids 5, der beispielsweise durch eine Füllstandsmarkierung vorbestimmt sein kann, in dem Aufnahmevolumen 3 angeordneten Luftraum 11 wirken kann. Die Plasmaquelle 7 ist hier an einer dem Aufnahmevolumen 3 zugeordneten Abdeckeinrichtung 13 angeordnet, insbesondere in die Abdeckeinrichtung 13 integriert. Die Abdeckeinrichtung 13 ist hier als Deckel für ein insbesondere becherartiges Aufnahmegefäß 15 ausgebildet, wobei das Aufnahmevolumen 3 in dem Aufnahmegefäß 15 angeordnet ist. Mittels der Abdeckeinrichtung 13 kann das Aufnahmegefäß 15 abgedeckt werden, wodurch das Aufnahmevolumen 3 als geschlossenes Volumen bereitgestellt wird.

Das hier dargestellte Ausführungsbeispiel der Vorrichtung 1 weist einen Sockel 17 auf, der eine Stellfläche 19 zur Anordnung des Aufnahmegefäßes 15 aufweist. Dabei ist bei dem hier dargestellten Ausführungsbeispiel eine nicht dargestellte Steuereinrichtung und/oder eine ebenfalls nicht dargestellte elektrische Speichereinrichtung zur Ansteuerung und/oder Versorgung der Plasmaquelle 7 in dem Sockel 17 angeordnet. Die Abdeckeinrichtung 13 und insbesondere die an oder in dieser angeordnete Plasmaquelle 7 ist/sind daher hier über eine elektrische Verbindungsleitung 21 mit dem Sockel 17 verbunden.

Es ist alternativ allerdings auch möglich, dass die Steuereinrichtung und/der die elektrische Speichereinrichtung - insbesondere gemeinsam mit der Plasmaquelle 7 - in der Abdeckeinrichtung 13 angeordnet ist/sind. In diesem Fall ist es auch möglich, dass auf den Sockel 17 verzichtet wird.

Im Rahmen einer bevorzugten Ausführungsform des Verfahrens zur Behandlung von Zahnprothesen wird bevorzugt das Reinigungsfluid 5 in dem Aufnahmegefäß 15 angeordnet. Das Aufnahmegefäß 15 wird danach mithilfe der Abdeckeinrichtung 13 geschlossen, und es wird ein nicht-thermisches Plasma in dem Luftraum 11 mittels der Plasmaquelle 7 erzeugt.

Die Vorrichtung 1 ist eingerichtet zur Durchmischung eines Plasmaerzeugnisses, insbesondere des nicht-thermischen Plasmas, mit dem Reinigungsfluid 5, um ein aktiviertes Reinigungsfluid zu erzeugen. Eine solche Durchmischung kann bei dem in Figur 1 dargestellten Ausführungsbeispiel insbesondere durch Schütteln des Aufnahmegefäßes 15 erfolgen, sodass wirksame Substanzen aus dem Plasma quasi in das Reinigungsfluid 5 ausgeschüttelt oder in diesem absorbiert werden, sodass das Reinigungsfluid aktiviert wird. Das Schütteln erfolgt bevorzugt im abgedeckten Zustand des Aufnahmegefäßes 15, wobei es möglich ist, dass zum Abdecken die Abdeckeinrichtung 13 verwendet wird. Es ist aber auch möglich, dass ein separater Deckel oder eine separate, zweite Abdeckeinrichtung ohne Plasmaquelle vorgesehen ist, sodass nach dem Erzeugen des Plasmas die erste Abdeckeinrichtung 13 entfernt und die separate, zweite Abdeckeinrichtung auf das Aufnahmegefäß 15 aufgesetzt wird. Das Aufnahmegefäß 15 kann dann geschüttelt werden, um das Reinigungsfluid zu aktivieren.

Die Vorrichtung 1 ist weiterhin eingerichtet zur Anwendung des aktivierten Reinigungsfluids auf wenigstens eine Zahnprothese und/oder wenigstens einen Zahn. Dabei ist es bei der hier dargestellten Vorrichtung 1 in einfacher Weise möglich, eine Zahnprothese in dem Aufnahmegefäß 15 anzuordnen, insbesondere in das aktivierte Reinigungsfluid einzulegen, sodass sie bevorzugt vollständig von dem aktivierten Reinigungsfluid bedeckt ist. Die Zahnprothese kann dann eine vorbestimmte Zeit in dem Aufnahmegefäß 15 ruhen und auf diese Weise gereinigt, insbesondere desinfiziert, und/oder gebleicht werden.

Das Aufnahmegefäß 15 ist bevorzugt nach Art eines Zahnbechers oder Zahnglases ausgebildet.

Es ist in nicht zur Erfindung gehörender Weise auch möglich, dass das aktivierte Reinigungsfluid im Mund eines Anwenders angewendet wird, beispielsweise durch Spülen oder Gurgeln. Da das aktivierte Reinigungsfluid bevorzugt Trinkwasserqualität aufweist, ist es dabei unschädlich, wenn es zumindest teilweise von dem Anwender geschluckt wird.

**Fig. 2** zeigt eine Detaildarstellung der Vorrichtung 1 gemäß Figur 1, wobei insbesondere die Plasmaquelle 7 näher erläutert wird. Gleiche und funktionsgleiche Elemente sind mit gleichen Bezugszeichen versehen, sodass insofern auf die vorangegangene Beschreibung verwiesen wird. Die Plasmaquelle 7 arbeitet hier nach dem Prinzip der dielektrischen Barriereentladung (dielectric barrier discharge - DBD). Dabei ist hier eine erste Elektrode 23 durch ein Dielektrikum 25, welches beispielsweise als Glasplatte ausgebildet sein kann, von einer zweiten Elektrode 27 beabstandet, wobei hier die Elektroden 23, 27 jeweils an gegenüberliegenden Oberflächen des Dielektrikums 25 angeordnet sind. Dabei ist die erste Elektrode 23 auf einer dem Luftraum 11 zugewandten Seite des Dielektrikums 25 angeordnet, wobei die zweite Elektrode 27 auf einer dem Luftraum 11 abgewandten Seite des Dielektrikums 25 angeordnet ist. Die erste Elektrode 23 kann auch in das Dieleketrikum 25 eingebettet sein.

Die erste Elektrode 23 ist bevorzugt als Gitterelektrode ausgebildet, die insbesondere aus einzelnen Leiterelementen gebildet sein kann, die gitterförmig über die Oberfläche des Dielektrikums 25 verteilt angeordnet sind.

Die zweite Elektrode 27 ist mit einer elektrischen Leistungsquelle 29, insbesondere einer Spannungsquelle, verbunden, insbesondere derart, dass ein von Masse verschiedenes Potential an die zweite Elektrode 27 anlegbar ist. Die Leistungsquelle 29 kann insbesondere als Wechselspannungsquelle ausgebildet sein, sodass eine Wechselspannung an die zweite Elektrode 27 anlegbar ist.

Die erste Elektrode 23 ist vorzugsweise mit einem Masseanschluss 31 verbunden. Dabei kann es sich um einen Masseanschluss 31 der Leistungsquelle 29, aber auch um einen von dieser externen Masseanschluss 31 handeln.

Auch eine umgekehrte Kontaktierung der Elektroden 23, 27 ist möglich, wobei jedoch die einem Benutzer zugewandte, erste Elektrode 23 aus Sicherheitsgründen bevorzugt auf Masse gelegt wird.

Die Leistungsquelle 29 ist bevorzugt Teil einer Steuereinrichtung 33 zur Ansteuerung der Plasmaquelle 7. Wird die Leistungsquelle 29 aktiviert und somit eine Spannung, insbesondere eine Wechselspannung, an der zweiten Elektrode 27 angelegt, wird in dem Luftraum 11 ein hier schematisch angedeutetes, nicht-thermisches Plasma 35 erzeugt.

Es ist möglich, dass beide Elektroden 23, 27 in das Dielektrikum 25 eingebettet und somit insbesondere gekapselt sind. Die Elektrodenanordnung der Plasmaquelle kann sehr dünn ausgebildet sein, wobei sie insbesondere eine Dicke oder Stärke von wenigstens 20 µm bis höchstens 500 µm, vorzugsweise bis höchstens 300 µm, aufweisen kann. Die Elektrodenanordnung ist somit sehr flexibel und kann ohne weiteres in eine für die Vorrichtung 1 günstige Form gebracht werden.

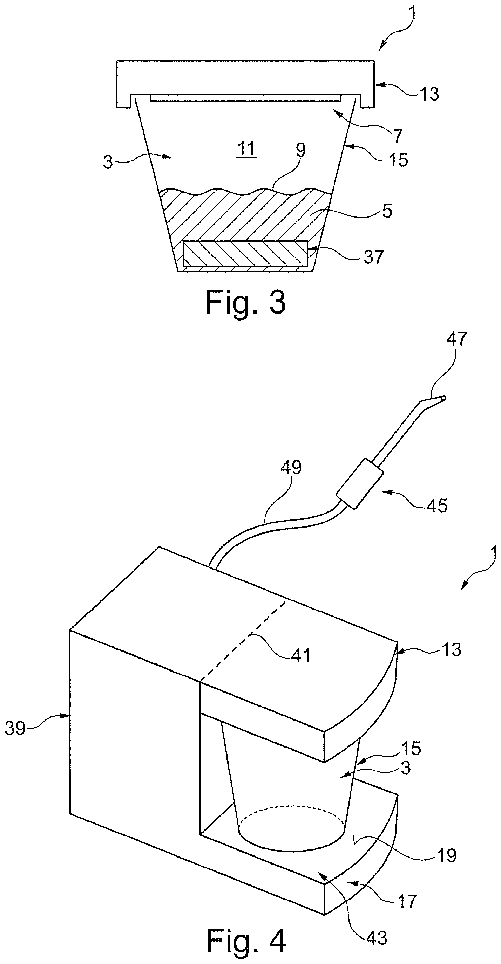

**Fig. 3** zeigt eine schematische Darstellung eines ersten Ausführungsbeispiels der Vorrichtung 1. Gleiche und funktionsgleiche Elemente sind mit gleichen Bezugszeichen versehen, sodass auf die vorangegangene Beschreibung verwiesen wird. Das hier dargestellte Ausführungsbeispiel weist keinen Sockel 17 auf. In diesem Fall sind die Steuereinrichtung 33 und vorzugsweise die elektrische Speichereinrichtung für die Plasmaquelle 7 bevorzugt in die Abdeckeinrichtung 13 integriert. Es ist aber auch möglich, dass auch dieses im Folgenden näher erläuterte Ausführungsbeispiel einen Sockel 17 aufweist und insoweit wie das in Figur 1 dargestellte Ausführungsbeispiel ausgebildet ist. Auch ist es möglich, dass bei dem Ausführungsbeispiel gemäß Figur 3 die Plasmaquelle 7 so ausgebildet ist, wie dies in Figur 2 dargestellt ist.

Bei dem in Figur 3 dargestellten Ausführungsbeispiel ist eine Mischeinrichtung 37 vorgesehen, die hier nur schematisch dargestellt ist. Die Mischeinrichtung 37 kann als Zerstäuber, insbesondere als Piezo- oder Ultraschallzerstäuber, oder aber als Belüftungseinrichtung, insbesondere als Aerator, ausgebildet sein. Ist die Mischeinrichtung 37 als Aerator ausgebildet, weist sie vorzugsweise eine poröse Keramik auf, durch welche plasmahaltige Luft aus dem Luftraum 11 in das Reinigungsfluid 5 eingeleitet werden kann. Hierzu ist vorzugsweise eine Fluidleitung zu dem Luftraum 11 vorgesehen, die in Figur 3 nicht dargestellt ist. Ist eine Mischeinrichtung 37 vorgesehen, bedarf es keines umständlichen Schüttelns des Aufnahmegefäßes 15 oder sonstiger Teile der Vorrichtung 1. Vielmehr kann das Durchmischen einfach, bequem und reproduzierbar mittels der Mischeinrichtung 37 erfolgen.

**Fig. 4** zeigt eine schematische Darstellung eines zweiten Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Bei dem vorliegenden Ausführungsbeispiel sind die Abdeckeinrichtung 13 und der Sockel 17 an einem gemeinsamen Grundkörper 39 der Vorrichtung 1 angeordnet, wobei die Steuereinrichtung und/oder die elektrische Speichereinrichtung für die Plasmaquelle 7 in dem Grundkörper 39 angeordnet oder in diesen integriert sein kann/können. Weiterhin kann eine gegebenenfalls für den Betrieb der Mischeinrichtung 37, die hier nicht explizit dargestellt ist, vorgesehene Einrichtung in dem Grundkörper 39 angeordnet oder in diesen integriert sein, beispielsweise eine Steuereinrichtung zum Betrieb eines Piezozerstäubers, oder eine Pumpe zur Förderung von plasmahaltigem Gas oder Luft zu einem Aerator. Der Sockel 17 kann insbesondere eine in die Stellfläche 19 integrierte Anschlusseinrichtung aufweisen, an welche eine Durchführung durch eine Wandung - insbesondere den Boden - des Aufnahmegefäßes 15 für einen Anschluss der Mischeinrichtung 37 angeschlossen werden kann, wenn das Aufnahmegefäß 15 auf der Stellfläche 19 angeordnet wird. Es kann sich bei der Durchführung um eine elektrische Durchführung und/oder eine Fluiddurchführung handeln. Plasmahaltige Luft oder plasmahaltiges Gas können aus dem Luftraum 11 auch über die Abdeckeinrichtung 13 entnommen werden, die dann bevorzugt eine entsprechende Saugöffnung sowie eine entsprechende Fluidleitung aufweist.

Bei dem hier dargestellten Ausführungsbeispiel der Vorrichtung 1 ist die Abdeckeinrichtung 13 verlagerbar an dem Grundkörper 39 angeordnet, hier nämlich insbesondere um eine Scharnierachse 41 schwenkbar. Die Abdeckeinrichtung 13 ist also insbesondere nach oben in eine Öffnungsposition und nach unten in eine Abdeckposition schwenkbar, wobei sie in der Abdeckposition das Aufnahmegefäß 15 abdeckt. Es ist alternativ auch möglich, dass die Abdeckeinrichtung 13 um eine schräg, insbesondere senkrecht zu der Scharnierachse 41 verlaufende Achse seitlich von der in Figur 4 dargestellten Abdeckposition in eine Öffnungsposition schwenkbar ist. Auch ist es möglich, dass die Abdeckeinrichtung 13 an dem Grundkörper 39 in anderer Weise verlagerbar, insbesondere auch linear verlagerbar, beispielsweise in Höhenrichtung, vorgesehen sein kann, um in eine Öffnungsposition einerseits und in eine Abdeckposition andererseits verlagerbar zu sein. Bevorzugt ist die Abdeckeinrichtung 13 derart verlagerbar an dem Grundkörper 39 angeordnet, dass sie in die Abdeckposition verlagert wird, wenn das Aufnahmegefäß 15 in einem hierfür vorgesehenen Anordnungsbereich 43 angeordnet wird. In diesem Fall ist die Abdeckeinrichtung 13 insbesondere automatisch schließbar, wobei sie bevorzugt auch automatisch offenbar, also in ihrer Öffnungsposition verlagerbar ist, wenn das Aufnahmegefäß 15 aus dem Anordnungsbereich 43 entnommen wird.

Besonders bevorzugt ist vorgesehen, dass die Plasmaquelle 7 automatisch aktivierbar ist, wenn die Abdeckeinrichtung 13 geschlossen wird, und insbesondere, wenn das Aufnahmegefäß 15 in dem Anordnungsbereich 43 angeordnet wird.

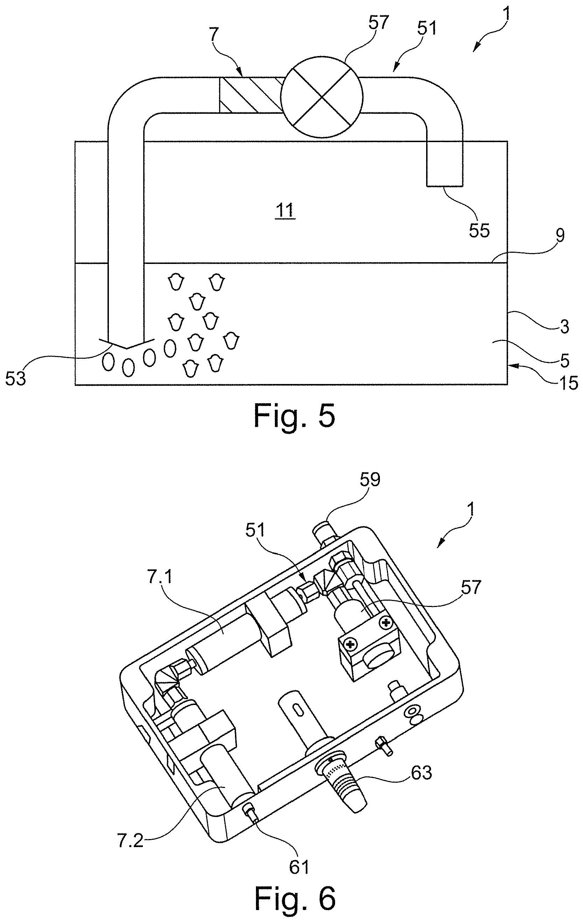

**Fig. 5** zeigt eine schematische Darstellung eines dritten Ausführungsbeispiels der Vorrichtung 1. Gleiche und funktionsgleiche Elemente sind mit gleichen Bezugszeichen versehen, sodass auf die vorangegangene Beschreibung verwiesen wird. Bei dem hier dargestellten Ausführungsbeispiel ist die Plasmaquelle 7 separat von dem Aufnahmevolumen 3 angeordnet, wobei sie insbesondere außerhalb des Aufnahmegefäßes 15 angeordnet ist. Die Plasmaquelle 7 ist insbesondere in einer Mediumleitung 51 angeordnet, wobei die Mediumleitung 51 zur Durchleitung eines gasförmigen Mediums ausgebildet ist. Die Plasmaquelle 7 ist derart angeordnet und eingerichtet, dass ein Plasma in dem durch die Mediumleitung 51 durchgeleiteten gasförmigen Medium durch die Plasmaquelle 7 erzeugbar ist. Die Mediumleitung 51 weist einen Medienauslass 53 auf, der in das Aufnahmevolumen 3 unterhalb des Füllstands 9, also submers in das Reinigungsfluid 5 mündet. Dabei ist es insbesondere möglich, dass der Medienauslass 53 in einen Belüfter oder Aerator mündet.

Die Mediumleitung 51 weist einen Medieneinlass 55 auf, der in den Luftraum 11 über dem bestimmungsgemäßen Füllstand 9 für das Reinigungsfluid 5 mündet. In der Mediumleitung 51 ist eine Fördereinrichtung 57 angeordnet, welche zur Förderung des gasförmigen Mediums entlang der Mediumleitung 51 von dem Medieneinlass 55 über die Plasmaquelle 7 zu dem Medienauslass 53 dient. Die Plasmaquelle 7 ist dabei stromabwärts des Medieneinlass 55 und stromaufwärts des Medienauslass 53 in der Mediumleitung 51 angeordnet. Die Fördereinrichtung 57 ist bei dem hier dargestellten Ausführungsbeispiel stromaufwärts der Plasmaquelle 7 angeordnet.

Die Plasmaquelle 7 kann eine Hohlelektrodenanordnung, insbesondere eine zylindrische oder auch im Querschnitt polygonale Elektrodenanordnung aufweisen, aber auch wenigstens eine planare Elektrodenanordnung, oder eine Stapelanordnung planarer Elektroden. Es ist auch möglich, dass die Plasmaquelle 7 eine perforierte Elektrodenanordnung aufweist, durch welche das gasförmige Medium hindurchgeleitet wird.

Das in Figur 5 dargestellte Ausführungsbeispiel kann bevorzugt derart mit dem in Figur 4 dargestellten Ausführungsbeispiel kombiniert werden, dass die Plasmaquelle 7 in oder an dem Grundkörper 39 angeordnet ist, wobei sich die Mediumleitung 51 bevorzugt durch den Grundkörper 39 oder entlang des Grundkörpers 39 erstreckt. Auch die Fördereinrichtung 57 ist vorzugsweise an oder in dem Grundkörper 39 angeordnet. Der Medieneinlass 55 kann bevorzugt in die Abdeckeinrichtung 13 integriert oder in der Abdeckeinrichtung 13 angeordnet sein. Der Medienauslass 53 kann wiederum bevorzugt an oder in dem Sockel 17 - insbesondere in Form einer Durchführung - angeordnet, insbesondere in den Sockel 17 integriert sein. Die Mediumleitung 51 kann dann insbesondere von der Abdeckeinrichtung 13 durch den Grundkörper 39 in den Sockel 17 führen.

**Fig. 6** zeigt eine Darstellung eines vierten Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Dabei ist hier insbesondere eine separat von dem Aufnahmevolumen 3 und insbesondere von dem Aufnahmegefäß 15 anordenbare Einheit dargestellt, welche die Plasmaquelle 7, die Fördereinrichtung 57 und teilweise die Mediumleitung 51 aufweist. Diese Einrichtung kann als separate Baugruppe vorgesehen sein. Sie weist hier einen ersten Leitungsverbinder 59 auf, der zur Verbindung mit einem Abschnitt der Mediumleitung 51 dient, welcher zu dem Medieneinlass 55 führt, sowie einen zweiten Leitungsverbinder 61, der zur Verbindung mit einem Abschnitt der Mediumleitung 51 dient, welcher zu dem Medienauslass 53 führt. Die Plasmaquelle 7 weist hier zwei zylindrische Elektrodenanordnungen 7.1, 7.2 auf. Dargestellt ist auch eine elektrische Durchführung 63, über welche die Elektrodenanordnungen 7.1, 7.2 kontaktierbar sind.

Mit den Ausgestaltungen der Vorrichtung 1 gemäß den Figuren 5 und 6 ist plasmahaltige Luft über den Luftraum 11, die Mediumleitung 51 und das Reinigungsfluid 5 im Kreislauf führbar, sodass besonders schnell ein aktiver Betriebszustand der Vorrichtung 1 herstellbar ist.

Bei allen bisher beschriebenen Ausführungsbeispielen der Vorrichtung 1 ist es möglich, dass das Aufnahmegefäß 15 eingerichtet ist zur Aufnahme von wenigstens einer Zahnprothese. Insbesondere - aber nicht ausschließlich - bei dem in Figur 4 dargestellten Ausführungsbeispiel ist es zusätzlich oder alternativ möglich, dass eine Applikationseinrichtung 45 vorgesehen ist, die zur Zuführung des aktivierten Reinigungsfluids in den Mund eines Anwenders eingerichtet ist. Hierzu kann die Applikationseinrichtung 45 insbesondere eine Munddüse 47 aufweisen. Die Vorrichtung 1 ist somit insgesamt bevorzugt als Munddusche ausgebildet. Die Applikationseinrichtung 45 weist bevorzugt eine Schlauchverbindung 49 auf, mit der sie insbesondere mit dem Grundkörper 39 verbunden ist. In dem Grundkörper 39 kann eine Pumpe oder dergleichen angeordnet sein, wobei mittels dieser Pumpe oder dergleichen aktiviertes Reinigungsfluid aus dem Aufnahmevolumen 3 durch die Schlauchverbindung 49 und die Munddüse 47 in den Mund eines Anwenders förderbar ist. Zur Entnahme des aktivierten Reinigungsfluids aus dem Aufnahmevolumen 3 kann insbesondere eine Fluiddurchführung in einer Wandung, insbesondere einem Boden des Aufnahmegefäßes 15 genutzt werden, die an eine entsprechende Anschlusseinrichtung oder Andockstelle in der Stellfläche 19 angeschlossen werden kann.

Bei einer bevorzugten Ausführungsform des hier vorgeschlagenen Verfahrens wird ein Verhältnis von einem Luftvolumen und/oder einer Luftmasse, in dem/der das nicht-thermische Plasma erzeugt wird, zu einem Volumen und/oder einer Masse des Reinigungsfluids 5 zu wenigstens 1:3 bis höchstens 3:1 gewählt.

Eine Einschaltdauer und/oder eine Leistung der Plasmaquelle 7 für die Erzeugung des nicht-thermischen Plasmas wird bevorzugt so gewählt, dass aktive Sauerstoffspezies oder aktive Stickstoffspezies in dem nicht-thermischen Plasma überwiegen.

Bevorzugt wird ein aktiviertes Reinigungsfluid erzeugt, welches einen Wasserstoffperoxidgehalt unterhalb eines zum Zeitrang der Anmeldung geltenden Grenzwerts in der europäischen Union für nicht-professionelles Zahnbleichen aufweist, und/oder welches Trinkwasserqualität aufweist.

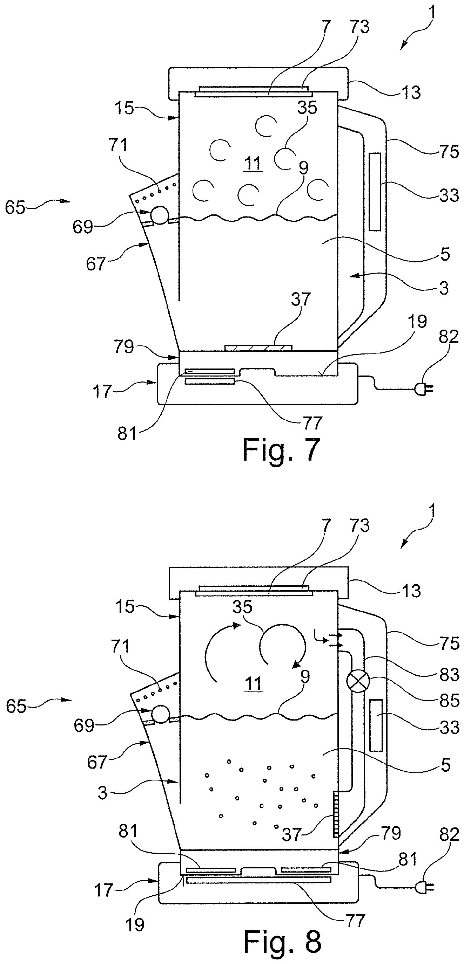

**Fig. 7** zeigt eine Darstellung eines zweiten, nicht zur Erfindung gehörenden Beispiels der Vorrichtung 1. Gleiche und funktionsgleiche Elemente sind mit gleichen Bezugszeichen versehen, sodass insofern auf die vorangegangene Beschreibung verwiesen wird. Die Vorrichtung 1 weist hier eine Gasabtrenneinrichtung 65 in Form einer an dem Aufnahmegefäß 15 angeordneten Tülle 67 auf, die submers, also unterhalb des Füllstands 9, in das Aufnahmevolumen 3 mündet. Somit ist es möglich, das aktivierte Reinigungsfluid auszugießen, ohne zugleich gasförmige Bestandteile, insbesondere Ozon, des Plasmaerzeugnisses zu entnehmen.

In die Tülle 67 ist ein Rückschlagventil in Form eines Ballventils 69 integriert. Ein Gitter 71 hindert die Absperrkugel des Ballventils 69 am Herausfallen und kann zugleich gegebenenfalls zur Filtration des Reinigungsfluids verwendet werden..

Die Vorrichtung 1 weist außerdem eine in die Abdeckeinrichtung 13 integrierte Heizeinrichtung 73 auf, die insbesondere zur Verhinderung unerwünschter Kondensation vorgesehen ist.

Das Aufnahmegefäß 15 weist einen Handgriff 75 auf, in den hier beispielsweise die Steuereinrichtung 33 sowie vorzugsweise eine Batterie oder dergleichen integriert ist/sind.

Die Mischeinrichtung 37 ist hier in nicht zur Erfindung gehörender Weise als Piezozerstäuber ausgebildet.

Der Sockel 17 weist eine induktive Spannungsversorgung 77 auf, wobei in einen Boden 79 des Aufnahmegefäßes eine Induktionsspule 81 integriert ist. Der induktiven Spannungsversorgung ist hier ein Netzanschlusstecker 82 zugeordnet.

**Fig. 8** zeigt eine Darstellung eines fünften Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Im Folgenden werden insbesondere Unterschiede zu dem sechsten Ausführungsbeispiel gemäß Figur 7 erläutert: Bei dem hier in Figur 8 dargestellten Ausführungsbeispiel ist in den Handgriff 75 eine Fluidleitung 83 integriert, mittels der plasmaaktivierte Luft - wie durch Pfeile angedeutet - aus dem Luftraum 11 entnehmbar und der hier als Aerator ausgebildeten Mischeinrichtung 37 zuführbar ist. Hierzu ist in der Fluidleitung 83 eine Fluid-Fördereinrichtung 85, vorzugsweise eine Membranpumpe angeordnet.

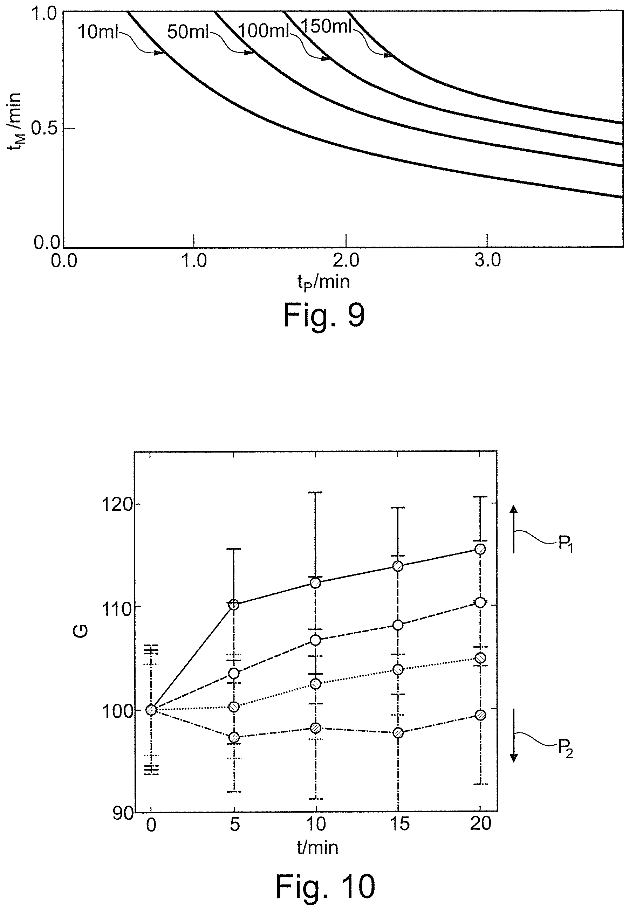

**Fig. 9** zeigt eine diagrammatische Darstellung der Effektivität des hier vorgeschlagenen Verfahrens. Dabei ist auf der horizontalen Achse eine Einschaltdauer t_{P} für die Plasmaquelle 7 in Minuten abgetragen. Auf der vertikalen Achse ist eine Mischungsdauer t_{M} in Minuten als Dauer der Durchmischung des Plasmaerzeugnisses mit dem Reinigungsfluid abgetragen. Weiterhin sind in dem Diagramm verschiedene Grenzkurven für verschiedene Volumina von Wasser als Reinigungsfluid angegeben, wobei die Volumina den Kurven jeweils durch Pfeile zugeordnet sind. In den Wasservolumina wurden jeweils Bakterien vom Typ Escherichia coli suspendiert. Anschließend wurde das Wasser in einem Aufnahmegefäß angeordnet. Die Plasmaquelle wurde für die angegebene Einschaltdauer t_{P} aktiviert, und das derart erzeugte Plasmaerzeugnis wurde für die Durchmischungszeit t_{M} mit dem Wasser vermischt. Die hier dargestellten Grenzkurven geben für jedes angegebene Volumen jeweils eine Grenze an, oberhalb und rechts von der ein Gebiet angeordnet ist, in welchem die Bakterienreduktion im Wasser mehr als 6 Größenordnungen betrug. Wird also die jeweilige Grenzlinie zu höheren Einschaltdauern der Plasmaquelle und/oder zu höheren Durchmischungszeiten hin überschritten, beträgt die Bakterienreduktion in dem Wasser durch die Plasmaaktivierung mehr als 6 Größenordnungen. Dies kann insbesondere als sogenannter Sterilisierungsvertrauensgrad (sterility assurance level - SAL) ausgedrückt werden, wobei ein SAL-Wert auch für die Bewertung eines Sterilisierungsprozesses verwendet werden kann. Eine Reduzierung um 6 Größenordnungen bedeutet eine Reduzierung der Bakterienbelastung um einen Faktor 10⁶, was üblicherweise im Sinne eines SAL-Werts auch als 6-log-Reduction bezeichnet wird.

Anhand von Figur 9 zeigt sich demnach, dass das hier vorgeschlagene Verfahren und die hier vorgeschlagene Vorrichtung äußerst effizient sind und in kurzer Zeit eine erhebliche Reduktion einer Bakterienbeladung leisten können. Eine ähnliche Darstellung kann für Viren deshalb nicht angegeben werden, weil sich hier in jedem Versuch eine Reduktion um mehr als 8 Größenordnungen zeigte, sodass innerhalb der gemessenen Zeiten quasi alle Viren inaktiviert waren.

**Fig. 10** zeigt eine diagrammatische Darstellung der Effektivität einer erfindungsgemäßen Ausführungsform des Verfahrens zum Bleichen von Gegenständen, insbesondere von Zahnprothesen. Dabei ist hier ein normalisierter Grauwert G eines Gegenstands, insbesondere einer Zahnprothese, aufgetragen gegen eine Behandlungszeit t in Minuten. Ein erster Pfeil P₁ weist in Richtung hellerer normalisierter Grauwerte. Ein zweiter Pfeil P₂ weist in Richtung dunklerer normalisierter Grauwerte. Eine erste, obere, durchgezogen dargestellte Kurve zeigt Ergebnisse einer Bleichbehandlung mit dem erfindungsgemäßen Verfahren unter Anwendung eines aktivierten Reinigungsfluids auf der Basis von Wasser mit einem Wasserstoffperoxid-Gehalt von weniger als 0,1 %. Eine zweite, unter der ersten Kurve dargestellte, gestrichelte Kurve zeigt Ergebnisse einer Bleichbehandlung mit einer 1 %igen, wässrigen Wasserstoffperoxid-Lösung. Eine dritte, unterhalb der zweiten Kurve angeordnete, punktierte Kurve zeigt Ergebnisse einer Behandlung mit nichtaktiviertem, kein Wasserstoffperoxid enthaltendem Wasser. Eine vierte, unterhalb der dritten Kurve dargestellte, strichpunktierte Kurve zeigt Kontrollwerte, die ohne jegliche Behandlung genommen sind. Die jeweils eingezeichneten Fehlerbalken zeigen die Bandbreite der Varianz in den verschiedenen Experimenten. Es zeigt sich klar, dass die Behandlung mit plasmaaktiviertem Wasser, wie in der ersten, durchgezogenen Kurve dargestellt, bei gleicher Behandlungsdauer eine deutlich bessere Bleichwirkung bereitstellt, als die Behandlung mit wässriger, 1 %iger Wasserstoffperoxid-Lösung gemäß der zweiten, gestrichelten Kurve.

Insgesamt zeigt sich, dass mit dem hier vorgeschlagenen Verfahren und der hier vorgeschlagenen Vorrichtung 1 eine ebenso einfache wie effiziente und zugleich in Hinblick auf die Gesundheit eines Anwenders unbedenkliche Möglichkeit geschaffen wird, insbesondere Zahnprothesen zu behandeln.

## Patentansprüche

1. Vorrichtung (1) zur Behandlung von Gegenständen, insbesondere von Zahnprothesen und/oder Zähnen, mit
- einem Aufnahmevolumen (3) zur Aufnahme eines Reinigungsfluids (5);
- einer Plasmaquelle (7), eingerichtet zur Erzeugung eines nicht-thermischen Plasmas (35), wobei
- die Vorrichtung (1) eingerichtet ist zur Durchmischung eines Plasmaerzeugnisses mit dem Reinigungsfluid (5), wodurch ein aktiviertes Reinigungsfluid erzeugbar ist, und wobei
- die Vorrichtung (1) eingerichtet ist zur Anwendung des aktivierten Reinigungsfluids auf einen Gegenstand, insbesondere auf wenigstens eine Zahnprothese und/oder wenigstens einen Zahn,
wobei die Plasmaquelle (7) angeordnet ist, um auf einen über einem Füllstand (9) des Reinigungsfluids (5) in dem Aufnahmevolumen (3) angeordneten Luftraum (11) zu wirken, wobei die Vorrichtung (1) eine Mischeinrichtung (37) zur Durchmischung des Plasmaerzeugnisses mit dem Reinigungsfluid (5) aufweist, wobei die Mischeinrichtung (37) in dem Aufnahmevolumen (3) angeordnet ist,
**dadurch gekennzeichnet, dass**
die Vorrichtung (1) eingerichtet ist, um einem Luftvolumen über einem Füllstand (9) des Reinigungsfluids (5) Gas oder Luft zu entnehmen und diese der Mischeinrichtung (37) in dem Aufnahmevolumen (3) unterhalb des Füllstands (9) zuzuführen.

2. Vorrichtung (1) nach Anspruch 1, **dadurch gekennzeichnet, dass** die Plasmaquelle (7) an einer dem Aufnahmevolumen (3) zugeordneten Abdeckeinrichtung (13) angeordnet oder in die Abdeckeinrichtung (13) integriert ist.

3. Vorrichtung (1) nach einem der vorhergehenden Ansprüche, **dadurch gekennzeichnet, dass** das Aufnahmevolumen (3) in einem Aufnahmegefäß (15) angeordnet ist.

4. Vorrichtung (1) nach einem der vorhergehenden Ansprüche, **dadurch gekennzeichnet, dass** die Plasmaquelle (7) separat von dem Aufnahmevolumen (3) angeordnet ist.

5. Vorrichtung (1) nach einem der vorhergehenden Ansprüche, **dadurch gekennzeichnet, dass** die Plasmaquelle (7) in einer Mediumleitung (51) zur Durchleitung eines gasförmigen Mediums angeordnet und eingerichtet ist, um ein Plasma in dem durch die Mediumleitung (51) durchgeführten gasförmigen Medium zu erzeugen, wobei die Mediumleitung (51) einen Medienauslasss (53) aufweist, der in das Aufnahmevolumen (3) für das Reinigungsfluid (5) mündet.

6. Vorrichtung (1) nach einem der vorhergehenden Ansprüche, **dadurch gekennzeichnet, dass** die Mediumleitung (51) einen Medieneinlass (55) aufweist, der in einen Luftraum (11) über einem bestimmungsgemäßen Füllstand des Reinigungsfluids (5) in dem Aufnahmevolumen (3) mündet.

7. Vorrichtung (1) nach einem der vorhergehenden Ansprüche, **dadurch gekennzeichnet, dass** die Vorrichtung (1) einen Sockel (17) aufweist, der eine Stellfläche (19) zur Anordnung des Aufnahmegefäßes (15) aufweist, wobei vorzugsweise eine Steuereinrichtung und/oder eine elektrische Speichereinrichtung für die Plasmaquelle (7)
a) in dem Sockel (17), und/oder
b) in einer dem Aufnahmevolumen (3) zugeordneten Abdeckeinrichtung (13)
angeordnet ist.

8. Vorrichtung (1) nach einem der vorhergehenden Ansprüche, **dadurch gekennzeichnet, dass**
a) das Aufnahmegefäß (15) eingerichtet ist zur Aufnahme von wenigstens einer Zahnprothese, und/oder
b) die Vorrichtung (1) eine Applikationseinrichtung (45) zur Zuführung des aktivierten Reinigungsfluids in den Mund eines Anwenders aufweist.

9. Vorrichtung (1) nach einem der vorhergehenden Ansprüche, **dadurch gekennzeichnet, dass** die Vorrichtung (1) einen Anordnungsbereich (43) zur Anordnung des Aufnahmegefäßes (15) aufweist, wobei vorzugsweise eine Abdeckeinrichtung (13) derart verlagerbar an einem Grundkörper (39) der Vorrichtung (1) angeordnet ist, dass die Abdeckeinrichtung (13) in eine das Aufnahmegefäß (15) abdeckende Abdeckposition verlagert wird, wenn das Aufnahmegefäß (15) in dem Anordnungsbereich (43) angeordnet wird.

10. Vorrichtung (1) nach einem der vorhergehenden Ansprüche, **dadurch gekennzeichnet,** die Mischeinrichtung (37)
a) eine Belüftungseinrichtung, insbesondere einen Aerator, und/oder
b) einen Medienauslass (53) einer die Plasmaquelle (7) aufweisenden Mediumleitung (51) aufweist.

11. Verfahren zum Behandeln, insbesondere Reinigen, von Gegenständen, insbesondere von Zahnprothesen, mit einer Vorrichtung gemäß einem der Ansprüche 1 bis 10 und mit folgenden Schritten:
- Erzeugen eines nicht-thermischen Plasmas (35);
- Mischen eines Plasmaerzeugnisses mit einem Reinigungsfluid (5);
- Aktivieren des Reinigungsfluids mit dem Plasmaerzeugnis, und
- Anwenden des aktivierten Reinigungsfluids auf einen Gegenstand, insbesondere auf wenigstens eine Zahnprothese, außerhalb des Mundes eines Anwenders.

12. Verfahren nach Anspruch 11, **dadurch gekennzeichnet, dass** ein Verhältnis von einem Luftvolumen, in dem das nicht-thermische Plasma erzeugt wird, zu einem Volumen des Reinigungsfluids (5) zu wenigstens 1:3 bis höchstens 3:1 gewählt wird.

13. Verfahren nach einem der Ansprüche 11 und 12, **dadurch gekennzeichnet, dass** eine Einschaltdauer und/oder eine Leistung einer Plasmaquelle (7) für die Erzeugung des nicht-thermischen Plasmas (35) so gewählt wird, dass
a) aktive Sauerstoffspezies, oder
b) aktive Stickstoffspezies
in dem Plasmaerzeugnis überwiegen.

14. Verfahren nach einem der Ansprüche 11 bis 13, **dadurch gekennzeichnet, dass** als Reinigungsfluid
a) Wasser, insbesondere Trinkwasser;
b) eine alkoholische Lösung;
c) sauerstoffangereichertes Wasser, und/oder
d) eine phosphatgepufferte Salzlösung
verwendet wird.

15. Verfahren nach einem der Ansprüche 11 bis 14, **dadurch gekennzeichnet, dass** ein aktiviertes Reinigungsfluid erzeugt wird, welches
a) einen Wasserstoffperoxidgehalt, der kleiner ist als ein in der Europäischen Union an dem den Zeitrang der vorliegenden Anmeldung bestimmenden Tag geltenden Grenzwert für nicht-professionelles Zahnbleichen, und/oder
b) Trinkwasserqualität
aufweist.

## Claims

1. Device (1) for treating objects, in particular dental prosthetics and/or teeth, having:
- a receiving volume (3) for receiving a cleaning fluid (5);
- a plasma source (7) configured to generate a nonthermal plasma (35), wherein
- the device (1) is configured to mix a plasma product with cleaning fluid (5), whereby an activated cleaning fluid can be generated, and wherein
- the device (1) is configured to use the activated cleaning fluid on an object, in particular on at least one dental prosthetic and/or at least one tooth,
wherein the plasma source (7) is arranged to act on an air space (11) arranged above a fill level (9) of the cleaning fluid (5) in the receiving volume (3), wherein the device (1) has a mixing apparatus (37) for mixing the plasma product with the cleaning fluid (5), wherein the mixing apparatus (37) is arranged in the receiving volume (3),
**characterized in that**
the device (1) is configured to take gas or air from an air volume above a fill level of the cleaning fluid and to supply the gas or the air to the mixing apparatus (37) in the receiving volume (3) below the fill level (9).

2. Device (1) according to claim 1, **characterized in that** the plasma source (7) is arranged on a covering apparatus (13) assigned to the receiving volume (3), or is integrated in the covering apparatus (13).

3. Device (1) according to one of the prior claims, **characterized in that** the receiving volume (3) is arranged in a receiving container (15).

4. Device (1) according to one of the prior claims, **characterized in that** the plasma source (7) is arranged separate from the receiving volume (3).

5. Device (1) according to one of the prior claims, **characterized in that** the plasma source (7) is arranged in a medium line (51) for conducting a gaseous medium in order to generate a plasma in the gaseous medium conveyed by the medium line (51), wherein the medium line (51) has a media outlet (53) that terminates in the receiving volume (3) for the cleaning fluid (5).

6. Device (1) according to one of the prior claims, **characterized in that** the medium line (51) has a media inlet (55) which terminates in an air space (11) above a relevant fill level of the cleaning fluid (5) in the receiving volume (3).

7. Device (1) according to one of the prior claims, **characterized in that** the device (1) has a base (17) which has a mounting surface (19) for arranging the receiving container (15), wherein preferably a control apparatus and/or an electrical storage apparatus for the plasma source (7) is arranged:
a) in the base (17) and/or
b) in a covering apparatus (13) assigned to the receiving volume (3).

8. Device (1) according to one of the prior claims, **characterized in that**:
a) the receiving container (15) is configured to receive at least one dental prosthesis, and/or
b) the device (1) has an application apparatus (45) for supplying the activated cleaning fluid into the mouth of a user.

9. Device (1) according to one of the prior claims, **characterized in that** the device (1) has an arrangement region (43) for arranging the receiving container (15), wherein preferably a covering apparatus (13) can be moved on a main body (39) of the device (1) so that the covering apparatus (13) is moved into a covering position covering the receiving container (15) when the covering container (15) is arranged in the arrangement region (43).

10. Device (1) according to one of the prior claims, **characterized in that**, wherein the mixing apparatus (37) preferably has:
a) a ventilating apparatus, in particular an aerator, and/or
b) a media outlet (53) of a medium line (51) having the plasma source (7).

11. Method for treating, in particular cleaning, objects, in particular dental prosthetics and/or teeth, using a device according to one of the claims 1 to 10 having the following steps:
- generation of a non-thermal plasma (35);
- mixing of a plasma product with a cleaning fluid (5);
- activation of the cleaning fluid with the plasma product, and
- usage of the activated cleaning fluid on an object, in particular on at least one dental prosthetic, outside the mouth of the user.

12. Method according to claim 11, **characterized in that** a ratio of an air volume in which the nonthermal plasma is generated to a volume of the cleaning fluid (5) is selected to be at least 1:3 to at most 3:1.

13. Method according to one of claims 11 and 12, **characterized in that** a duty cycle and or a performance of a plasma source (7) for generating the nonthermal plasma (35) is selected so that:
a) active oxygen species or
b) active nitrogen species
predominate in the plasma product.

14. Method according to one of claims 11 to 13, **characterized in that**:
a) water, in particular drinking water;
b) an alcoholic solution;
c) oxygen-enriched water, and/or
d) a phosphate-buffered saline solution
is used as the cleaning fluid.

15. Method according to one of claims 11 to 14, **characterized in that** an activated cleaning fluid is generated that has:
a) a hydrogen peroxide content less than that of a threshold for nonprofessional dental whiteners applicable in the European Union on the date determining the priority of the present application, and/or
b) the quality of drinking water.

## Revendications

1. Dispositif (1) pour le traitement d'objets, en particulier de prothèses dentaires et/ou de dents, comprenant
- un volume de réception (3) pour recevoir un fluide de nettoyage (5) ;
- une source de plasma (7), agencée pour produire un plasma non thermique (35), dans lequel
- le dispositif (1) est conçu pour mélanger un produit de plasma avec le fluide de nettoyage (5), ce qui permet de produire un fluide de nettoyage activé, et dans lequel
- le dispositif (1) est conçu pour appliquer le fluide de nettoyage activé sur un objet, en particulier sur au moins une prothèse dentaire et/ou au moins une dent,
dans lequel la source de plasma (7) est agencée pour agir sur un espace d'air (11) disposé au-dessus d'un niveau de remplissage (9) du fluide de nettoyage (5) dans le volume de réception (3), le dispositif (1) présentant un dispositif de mélange (37) pour mélanger le produit de plasma avec le fluide de nettoyage (5), le dispositif de mélange (37) étant disposé dans le volume de réception (3),
**caractérisé en ce que**
le dispositif (1) est conçu pour prélever du gaz ou de l'air dans un volume d'air au-dessus d'un niveau de remplissage (9) du fluide de nettoyage (5) et pour l'amener au dispositif de mélange (37) dans le volume de réception (3) en dessous du niveau de remplissage (9).

2. Dispositif (1) selon la revendication 1, **caractérisé en ce que** la source de plasma (7) est disposée sur un dispositif de recouvrement (13) associé au volume de réception (3) ou est intégrée dans le dispositif de recouvrement (13).

3. Dispositif (1) selon l'une des revendications précédentes, **caractérisé en ce que** le volume de réception (3) est disposé dans un récipient de réception (15).

4. Dispositif (1) selon l'une des revendications précédentes, **caractérisé en ce que** la source de plasma (7) est disposée séparément du volume de réception (3).

5. Dispositif (1) selon l'une des revendications précédentes, **caractérisé en ce que** la source de plasma (7) est disposée dans une conduite de fluide (51) pour le passage d'un fluide gazeux et est conçue pour produire un plasma dans le fluide gazeux passant dans la conduite de fluide (51), la conduite de fluide (51) comportant une sortie de fluide (53) débouchant dans le volume de réception (3) du fluide de nettoyage (5).

6. Dispositif (1) selon l'une des revendications précédentes, **caractérisé en ce que** la conduite de fluide (51) présente une entrée de fluide (55) qui débouche dans un espace d'air (11) au-dessus d'un niveau de remplissage conforme du fluide de nettoyage (5) dans le volume de réception (3).

7. Dispositif (1) selon l'une des revendications précédentes, **caractérisé en ce que** le dispositif (1) comporte un socle (17) présentant une surface de pose (19) pour disposer le récipient de réception (15), un dispositif de commande et/ou un dispositif de stockage électrique pour la source de plasma (7) étant disposé(s) de préférence
a) dans le socle (17), et/ou
b) dans un dispositif de recouvrement (13) associé au volume de réception (3).

8. Dispositif (1) selon l'une des revendications précédentes, **caractérisé en ce que**
a) le récipient de réception (15) est conçu pour recevoir au moins une prothèse dentaire, et/ou
b) le dispositif (1) comporte un dispositif d'application (45) pour amener le fluide de nettoyage activé dans la bouche d'un utilisateur.

9. Dispositif (1) selon l'une des revendications précédentes, **caractérisé en ce que** le dispositif (1) présente une zone de disposition (43) pour disposer le récipient de réception (15), un dispositif de recouvrement (13) étant de préférence disposé de manière déplaçable sur un corps de base (39) du dispositif (1) de telle sorte que le dispositif de recouvrement (13) est déplacé dans une position de recouvrement recouvrant le récipient de réception (15) lorsque le récipient de réception (15) est disposé dans la zone de disposition (43).

10. Dispositif (1) selon l'une des revendications précédentes, **caractérisé en ce que** le dispositif de mélange (37) comporte
a) un dispositif d'aération, en particulier un aérateur, et/ou
b) une sortie de fluide (53) d'une conduite de fluide (51) présentant la source de plasma (7).

11. Procédé de traitement, notamment de nettoyage, d'objets, notamment de prothèses dentaires, avec un dispositif selon l'une des revendications 1 à 10 et comprenant les étapes suivantes :
- Production d'un plasma non thermique (35) ;
- Mélanger un produit de plasma avec un fluide de nettoyage (5) ;
- Activation du fluide de nettoyage avec le produit de plasma, et
- Appliquer le fluide de nettoyage activé sur un objet, en particulier sur au moins une prothèse dentaire, à l'extérieur de la bouche d'un utilisateur.

12. Procédé selon la revendication 11, **caractérisé en ce qu'**un rapport entre un volume d'air, dans lequel le plasma non thermique est produire, et un volume de fluide de nettoyage (5) est choisi entre au moins 1:3 et au plus 3:1.

13. Procédé selon l'une des revendications 11 et 12, **caractérisé en ce qu'**une durée de fonctionnement et/ou une puissance d'une source de plasma (7) pour la production du plasma non thermique (35) est choisie de telle sorte que
a) des espèces actives d'oxygène, ou
b) des espèces actives d'azote
prédominent dans le produit de plasma.

14. Procédé selon l'une des revendications 11 à 13, **caractérisé en ce que**, comme fluide de nettoyage
a) de l'eau, en particulier de l'eau potable ;
b) une solution alcoolique ;
c) de l'eau enrichie en oxygène, et/ou
d) une solution saline tamponnée au phosphate
est utilisée.

15. Procédé selon l'une des revendications 11 à 14, **caractérisé en ce qu'**un fluide de nettoyage activé est produit, lequel présente
a) une teneur en peroxyde d'hydrogène inférieure à une valeur limite pour le blanchiment des dents non professionnel en vigueur dans l'Union Européenne à la date déterminant la date de la présente demande, et/ou
b) qualité eau potable.
